# EUROPEAN PATENT APPLICATION

(11) **EP 3 878 958 A1**
(43) Date of publication of application: **15.09.2021**
(21) Application number: 20000105.5
(22) Date of filing: 11.03.2020
(51) Int. Cl.: C12N 9/22, C12N 15/55, C12N 15/10, C12N 15/82

(54) **CRISPR-CAS NUCLEASES FROM CPR-ENRICHED METAGENOME**

(71) Applicant: B.R.A.I.N. Biotechnology Research And Information Network AG, 64673 Zwingenberg (DE)
(72) Inventor: Scholz, Paul, 50931 Köln (DE); Pul, Uemit, 47259 Duisburg (DE); Zureck, Christian, 64653 Lorsch (DE)

(57) **Abstract**

The present invention relates to a nucleic acid molecule encoding an RNA-guided DNA endonuclease, which is (a) a nucleic acid molecule encoding the RNA-guided DNA endonuclease comprising or consisting of the amino acid sequence of SEQ ID NO: 1 or 3; (b) a nucleic acid molecule comprising or consisting of the nucleotide sequence of SEQ ID NO: 2 or 4; (c) a nucleic acid molecule encoding a RNA-guided DNA endonuclease the amino acid sequence of which is at least 70 % identical to the amino acid sequence of (a); preferably at least 80 % identical, more preferably at least 90 % identical, and most preferred at least 95% identical; (d) a nucleic acid molecule comprising or consisting of a nucleotide sequence which is at least 70 % identical to the nucleotide sequence of (b), preferably at least 80 % identical, more preferably at least 90 % identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

## Description

The present invention relates to a nucleic acid molecule encoding an RNA-guided DNA endonuclease, which is (a) a nucleic acid molecule encoding the RNA-guided DNA endonuclease comprising or consisting of the amino acid sequence of SEQ ID NO: 1 or 3; (b) a nucleic acid molecule comprising or consisting of the nucleotide sequence of SEQ ID NO: 2 or 4; (c) a nucleic acid molecule encoding a RNA-guided DNA endonuclease the amino acid sequence of which is at least 70 % identical to the amino acid sequence of (a); preferably at least 80 % identical, more preferably at least 90 % identical, and most preferred at least 95% identical; (d) a nucleic acid molecule comprising or consisting of a nucleotide sequence which is at least 70 % identical to the nucleotide sequence of (b), preferably at least 80 % identical, more preferably at least 90 % identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

CRISPR-Cas systems are widespread adaptive immunity systems of prokaryotes against invading foreign nucleic acids. So far, more than 30 different CRISPR-Cas systems have been identified that differ in their loci architecture, number, and identity of their genes encoding for the Cas (CRISPR-associated) proteins.

The typical signature of the CRISPR systems in prokaryotic genomes is the presence of short (30-45 bp) repetitive sequences (repeats) that are intervened by variable sequences (spacers) of similar lengths. The Cas proteins are located either upstream or downstream of the repeat-spacer cluster. According to their gene composition and mechanistic differences, the subtypes are classified into two CRISPR classes (Class 1 and 2). One of their major differences is that Class 1 CRISPR systems need a complex of multiple Cas proteins to degrade DNA, whereas Class 2 Cas proteins are single, large multidomain nucleases. The sequence-specificity of the Class 2 Cas proteins can simply be modified by synthetic CRISPR RNAs (crRNAs) in order to introduce targeted double-stranded DNA breaks. Most prominent members of such Class 2 Cas proteins are Cas9, Cpf1 (Cas12a) and Cms1, which are harnessed for genome-editing and successfully applied in many eukaryotic organisms including fungi, plants and mammalian cells. Whereas Cas9 and its orthologs are Class2 type II CRISPR nucleases, Cpf1 (WO2016/205711 BROAD Inst.; WO2017/141173 Benson Hill) and Cms1 (WO2019/030695 Benson Hill) belong to Class 2 type V nucleases. Cms1 and Cpf1 CRISPR nucleases are a class of CRISPR nucleases that have certain desirable properties compared to other CRISPR nucleases such as type II nucleases. For instance, in contrast to Cas9 nucleases Cms1 and Cpf1 do not require a trans-activating crRNA (tracrRNA), which is partially complementary to the precursor crRNA (pre-crRNA) (Deltcheva et al. (2011), Nature, 471(7340):602-607). Base-pairing of tracrRNA and pre-crRNA forms a Cas9-bound RNA:RNA duplex, which gets processed by RNase III and other unidentified nucleases. This mature tracrRNA:crRNA duplex mediates the target DNA recognition and cleavage by Cas9. In contrast, type V nucleases can process the pre-crRNA without the need for tracrRNA or cellular nucleases (like RNase III), which significantly simplifies the application of type V nucleases for (multiplex) genome editing.

Several new Class 2 proteins, like C2c1 (Cas12b), C2c2 (Cas13a) and C2c3 (Cas12c) have been identified in the genomes of cultivated bacteria or public available metagenomics datasets, e.g. gut metagenome (Shmakov et al. (2015), Mol Cell,60(3):385-97). According to the recent classification of CRISPR-Cas systems, Class 2 comprises 3 types and 17 subtypes (Makarova et al. (2020), Nat Rev Microbiol, 18(2):67-83).

Moreover, in a recent publication two new Class 2 proteins were discovered (CasX (Cas12a) and CasY (Cas12d)) in uncultivated prokaryotes by metagenome sequencing (Burstein et al. (2017), Nature, 542:237-241), indicating the presence of untapped Cas proteins from organisms which are not cultivated and/or identified yet.

A promising approach for the discovery of novel Class 2 Cas proteins is therefore to access the metagenomics resource by next generation sequencing selected environmental DNA (e.g. 1 cm³ forest soil contains ^{∼} 2.5 x 10¹⁰ bp DNA or ^{∼} 20 million genes) and computational identification of CRISPR-Cas systems (for example, Lei and Sun (2016), Bioinformatics, 32(17):i520-i528).

As discussed, the known CRISPR-Cas systems display certain differences as regards their mode of action. These molecular differences not only enlarge the possibilities of using the CRISPR-Cas system for genome editing in a broad range of different genetic backgrounds but also to circumvent issues of particular Cas nucleases when applied in certain organisms, e.g. pre-existing immune response to Cas9 in humans (Charlesworth et al. (2019), Nat Med, 25(2):249-254). Therefore, the identification of Cas nucleases from bacterial species with less direct contact to higher eukaryotes is of particular importance. It can be assumed that a number of yet undiscovered CRISPR-Cas systems exist with yet unknown characteristics, in particular in metagenome resources. Hence, although already several different CRISPR-Cas systems are known from the prior art there is an ongoing need to identify further CRISPR-Cas systems and in particular the RNA-guided DNA endonucleases of these systems. This need is addressed by the present invention

Accordingly, the present invention relates in first aspect to a nucleic acid molecule encoding an RNA-guided DNA endonuclease, which is (a) a nucleic acid molecule encoding the RNA-guided DNA endonuclease comprising or consisting of the amino acid sequence of SEQ ID NO: 1 or 3; (b) a nucleic acid molecule comprising or consisting of the nucleotide sequence of SEQ ID NO: 2 or 4; (c) a nucleic acid molecule encoding a RNA-guided DNA endonuclease the amino acid sequence of which is at least 70 % identical to the amino acid sequence of (a); preferably at least 80 % identical, more preferably at least 90 % identical, and most preferred at least 95% identical; (d) a nucleic acid molecule comprising or consisting of a nucleotide sequence which is at least 70 % identical to the nucleotide sequence of (b), preferably at least 80 % identical, more preferably at least 90 % identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

SEQ ID NOs 1 and 3 are the amino acid sequences of the novel CRISPR-Cas endonucleases Cps1 and Cps2, respectively, wherein Cps is an abbreviation of Cas-candidate Phyla Radiation Species. The novel CRISPR-Cas endonucleases Cps1 and Cps2 are encoded by the nucleotide sequences of SEQ ID NOs 2 and 4, respectively.

In accordance with the present invention the term "nucleic acid molecule" defines a linear molecular chain of nucleotides. The nucleic acid molecules according to the present invention consist of at least 2174 nucleotides. The group of molecules designated herein as "nucleic acid molecules" also comprises complete genes. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The term "polypeptide" as used herein interchangeably with the term "protein" describes linear molecular chains of amino acids, including single chain proteins or their fragments. The polypeptides / proteins according to the present invention contain at least 724 amino acids. Polypeptides may further form oligomers consisting of at least two identical or different molecules. The corresponding higher order structures of such multimers are, correspondingly, termed homo- or heterodimers, homo- or heterotrimers etc.. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is affected e.g. by glycosylation, acetylation, phosphorylation, ubiquitinylation and similar modifications which are well known in the art.

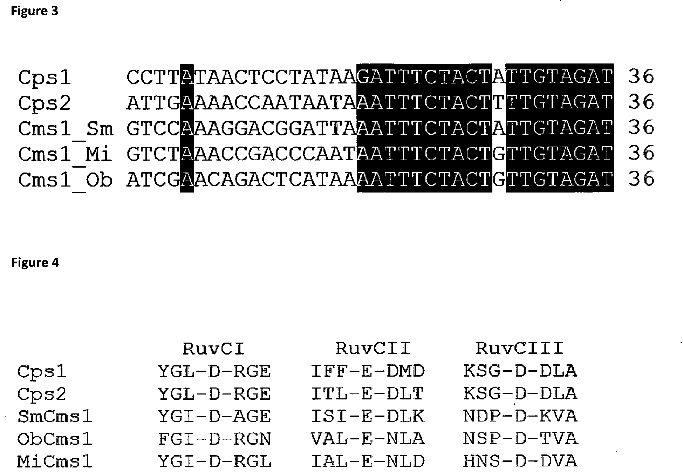

The term "RNA-guided DNA endonuclease" or "CRISPR(-Cas) endonuclease" describes an enzyme having the capability of cleaving the phosphodiester bond within a deoxyribonucleotide (DNA) strand thereby producing a double-strand break (DSB). Cps1 and Cps2 are classified as novel type V class 2 CRISPR nucleases which are known to introduce a staggered cut with a 5' overhang. Hence, an RNA-guided DNA endonuclease comprises an endonuclease domain, in particular a RuvC domain. The RuvC domain of Cps1 and Cps2 comprises three split RuvC motifs (RuvC I-III, Fig. 4; SEQ ID NOs 12 to 14 and 15 to 17, respectively). A RNA-guided DNA endonuclease also comprises a domain being capable of binding to a crRNA, also known as guide RNA (gRNA; also being designated DNA-targeting RNA herein).

The cleavage site of the RNA-guided DNA endonuclease is guided by a guide RNA. The gRNA confers the target sequence specificity to the RNA-guided DNA endonuclease. Such gRNAs are non-coding short RNA sequences which binds to the complementary target DNA sequences. The gRNA first binds to the RNA-guided DNA endonuclease by a binding domain that can interact with the RNA-guided DNA endonuclease. The binding domain that can interact with the RNA-guided DNA endonuclease typically comprises a region with a stem-loop structure. This stem-loop preferably comprises the sequence UCUACN₃₋₅GUAGAU (SEQ ID NO: 27, 35 and 36), with "UCUAC" and "GUAGA" base-pairing to form the stem of the stem-loop. N₃₋₅ denotes that any base may be present at this location, and 3, 4, or 5 nucleotides may be included at this location. The stem-loop most preferably comprises the stem loop direct repeat sequence of Cps1 or Cps2 as shown in Fig. 3 (SEQ ID NOs 28 and 29, respectively), but in the form of RNA (i.e. wherein T is replaced by U). The gRNA sequence guides the complex (known as CRISPR ribonucleoprotein (RNP) complex of the gRNA and the RNA-guided DNA endonuclease) via pairing to a specific location on a DNA strand, where the RNA-guided DNA endonuclease performs its endonuclease activity by cutting the DNA strand at the target site. The genomic target site of the gRNA can be any about 20 (typically 17 to 26) nucleotide DNA sequence, provided it meets two conditions: (i) The sequence is unique compared to the rest of the genome, and (ii) the target is present immediately adjacent to a Protospacer Adjacent Motif (PAM).

The cleavage site of the RNA-guided DNA endonuclease is, thus, furthermore defined by a PAM. The PAM is a short DNA sequence (usually 2-6 base pairs in length) that follows the DNA region targeted for cleavage by the CRISPR system. The exact sequence depends on which CRISPR endonuclease is used. CRISPR endonucleases and their respective PAM sequences are known in the art (see https://www.addgene.org/crispr/guide/#pam-table). For instance, the PAM being recognized by the first identified RNA-guided DNA endonuclease Cas9 is 5'-NGG-3' (where "N" can be any nucleotide base). The PAM is required for a RNA-guided DNA endonuclease to cut. In Cas9 it is found about 2-6 nucleotides upstream or downstream of the DNA sequence targeted by the guide RNA and 3-20 nucleotides upstream or downstream from the cut site. In type V systems (including Cps1 and Cps2) the PAM is located upstream of both, the target sequence and the cleavage site. The complex of the RNA-guided DNA endonuclease and the guide RNA comprises a so-called PAM interacting domain (Andres et al. (2014), Nature, 513(7519):569-573). Hence, the genomic locations that can be targeted for editing by an RNA-guided DNA endonuclease are limited by the presence and locations of the nuclease-specific PAM sequence. As Cps1 and Cps2 belong to the group of Type V Class 2 CRISPR nucleases a T rich PAM site was predicted. To validate this prediction a TTTN PAM site was used for the functional characterization of Cps1 and Cps2.

The term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence as well as nucleotide sequence analysis and alignments in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

As defined herein above, amino acid sequence and nucleotide sequence identities of at least 70%, at least 80%, at least 90% and at least 95% are envisaged by the invention. Furthermore, are envisaged with increasing preference amino acid sequence identities of at least 99.5 %, at least 99.8 %, and 99.9% identity by the invention.

With respect to these amino acid sequences and the amino acid sequences being encoded by these nucleotide sequences it is preferred that they maintain or essentially maintain the RNA-guided DNA endonuclease activity of SEQ ID NOs 1 and 3 of the invention. Hence, what is maintained or essentially maintained is the capability to bind to the gRNA to form a complex being capable of binding to the DNA target site of interest, where the endonuclease activity induces a DSB.

The maintenance or essentially maintenance of the RNA-guided DNA endonuclease activity can be analysed in a CRISPR-Cas genome editing experiment, for example, as is illustrated in Example 3. It is preferred that the amino acid sequences comprise and the nucleotide sequences encode a RuvC domain as shown in Figure 4, preferably the RuvC domain of SEQ ID NOs 12 to 14 or 15 to 17. As mentioned, the RuvC domain is an endonuclease domain.

The term "degenerate" designates the degeneracy of the genetic code. As is well known, the codons encoding one amino acid may differ in any of their three positions; however, more often than not, this difference is in the second or third position. For instance, the amino acid glutamic acid is specified by GAA and GAG codons (difference in the third position); the amino acid leucine is specified by UUA, UUG, CUU, CUC, CUA, CUG codons (difference in the first or third position); and the amino acid serine is specified by UCA, UCG, UCC, UCU, AGU, AGC (difference in the first, second, or third position).

As can be taken from the appended examples Cps1 and Cps2 were isolated from metagenome samples and in particular metagenome samples comprising candidate phyla radiation (CPR). Candidate phyla are bacterial phyla which are difficult to cultivate. It is estimated that candidate phyla represent over 15% of all bacterial diversity and may consist of more than 70 different phyla (Hug et al. (2016), Nature Microbiology; 1(5):16048). Hence, it was only possible to identify Cps1 and Cps2 as novel CRISPR-Cas endonucleases by generating a metagenome being enriched in candidate phyla and next generation sequences as illustrated in Examples 1 and 2. As a result of this innovative strategy the two novel endonucleases Cps1 and Cps2 were identified which are to the best knowledge of the inventors significantly distinct from all known CRISPR-Cas endonucleases. The amino acid sequences of Cps1 (SEQ ID NO: 1) and Cps2 (SEQ ID NO: 3) only share 42% and 48%, respectively, with the amino acid sequence of the Cms1-type CRISPR-Cas endonuclease of SEQ ID NO: 51 as described in US 2019/0048357 (See Fig. 1). To the best knowledge of the inventors this sequence of US 2019/0048357 is the "nearest neighbour" of Cps1 and Cps2 among the known CRISPR-Cas endonucleases.

As a proof of principle Example 3 shows that Cps1 is an active CRISPR-Cas endonuclease that can be successfully used to for genome editing. In Example 3 a mutant beta-glucuronidase gene is repaired in wheat embryo by using Cps1, a gRNA and a repair substrate.

Just as the type V CRISPR endonucleases Cms1 and Cpf1, both Cps1 and Cps2 do not require a trans-activating crRNA (tracrRNA). Moreover, the Cps1 and Cps2 containing CRISPR systems identified in the present application contain CRISPR repeat sequences with an RNA stem loop at 3'-ends of each repeat that is conserved in crRNAs of Cpf1 and Cms1 protein families. As discussed, the "nearest neighbour" of Cps1 and Cps2 among the known CRISPR-Cas endonucleases is a Cms1 polypeptide and each, Cps1 and Cps2 contain three short stretches of amino acids that are known to form the RuvC nuclease domain of Class 2 type V Cas proteins; see Fig. 3 and 4. For this reason Cps1 and Cps2 can be classified as novel Class 2 type V nucleases with overall no significant sequence identity to the known collection of Class 1 and Class 2 CRISPR-Cas endonucleases and with overall low sequence identity to a single Cms1-type endonuclease.

Since Cps1 and Cps2 are novel CRISPR-Cas endonucleases which are significantly distinct from the known collection of CRISPR-Cas endonucleases, Cps1 and Cps2 expand the known collection of CRISPR-Cas endonucleases applicable for genome editing, gene regulation and nucleic acid enrichment/purification in different biotechnological and pharmaceutical sectors. The sequence diversity of Cps1 and Cps2 at least strongly indicates that Cps1 and Cps2 are not only novel effector proteins with distinct locus architectures but also may display in certain aspects new molecular genome editing mechanisms.

In accordance with a preferred embodiment of the first aspect of the invention the nucleic acid molecule is operably linked to a promoter that is native or heterologous to the nucleic acid molecule.

A promoter is a region of DNA that leads to initiation of transcription of a particular gene. Promoters are generally located near the transcription start sites of genes, upstream on the DNA (towards the 5' region of the sense strand). Promoters are typically 100-1000 base pairs long. For transcription to take place, the enzyme that synthesizes RNA, known as RNA polymerase, must attach to the DNA near a gene. Promoters contain specific DNA sequences such as response elements that provide a secure initial binding site for RNA polymerase and for proteins called transcription factors that recruit RNA polymerase. Hence, the binding of the RNA polymerase and transcription factors to the promoter site ensures the transcription of the gene.

In this connection the term "operably linked" defines that the promoter is linked to the gene of the same DNA strand, such that upon binding of the RNA polymerase and transcription factors the transcription of the gene is initiated. Generally each gene is operably linked in its natural environment of the genome of a living organism to a promoter. This promoter is designated "natural promoter" or "wild-type promoter" herein. A heterologous promoter is distinct from the natural promoter or wild-type promoter. Hence, a nucleic acid molecule which is operably linked to a promoter that is heterologous to the nucleic acid molecule does not occur in nature.

Heterologous promoters that can be used to express a desired gene are known in the art and can, for example, be obtained from the EPD (eukaryotic promoter database) or EDPnew (https://epd.epfl.ch//index.php). In this database eukaryotic promoters including animal, plant and yeast promoters can be found.

The promoter can, for example, be a constitutively active, inducible, tissue-specific, or developmental stage-specific promoter. By using such promoter the desired timing and site of expression can be regulated.

The *AOX1* or *GAL1* promoter in yeast or the CMV- (Cytomegalovirus), SV40-, RSV-promoter (Rous sarcoma virus), chicken beta-actin promoters, CAG-promoter (a combination of chicken beta-actin promoter and cytomegalovirus immediate-early enhancer), the gai10 promoter, human elongation factor 1α-promoter, CaM-kinase promoter, and the Autographa californica multiple nuclear polyhedrosis virus (AcMNPV) polyhedral promoter are examples of constitutively active promoters.

Examples of inducible promoters are the Adhl promoter which is inducible by hypoxia or cold stress, the Hsp70 promoter which is inducible by heat stress, the PPDK promoter and the pepcarboxylase promoter which are both inducible by light. Also useful are promoters which are chemically inducible, such as the ln2-2 promoter which is safener induced (US 5,364,780), the ERE promoter which is estrogen induced, and the Axigl promoter which is auxin induced and tapetum specific but also active in callus (WO03060123 A1).

A tissue-specific promoter is a promoter that initiates transcription only in certain tissues. A developmental stage-specific promoter is a promoter that initiates transcription only in a certain developmental stage.

In the examples herein below the promoter of wheat U6 polymerase III has been used. Therefore the use of U6 promoter and in particular a U6 polymerase III promoter from wheat is preferred.

In accordance with a further preferred embodiment of the first aspect of the invention the nucleic acid molecule is linked to a nucleic acid sequence encoding a nuclear localization signal (NLS).

Further details on NLS will be provided herein below.

In accordance with another preferred embodiment of the first aspect of the invention said nucleic acid molecule is codon-optimized for expression in a eukaryotic cell, preferably a plant cell or an animal cell.

As discussed, Cps1 and Cps2 were isolated form bacterial metagenome samples. Hence, if Cps1 and Cps2 are to be expressed in eukaryotic cells they are expressed in a heterologous host cell or organism.

The gene encoding Cps1 or Cps2 polypeptide, respectively, can be codon-optimized for expression in the target cell, and can optionally include a sequence encoding an NLS and/or a peptide tag, such a purification tag. Further details on the tag will be provided herein below.

Codon optimization is a process used to improve gene expression and increase the translational efficiency of a gene of interest by accommodating codon bias of the host cell. A "codon-optimized gene" is therefore a gene having its frequency of codon usage designed to mimic the frequency of preferred codon usage of the host cell. Nucleic acid molecules can be codon optimized, either wholly or in part. Because any one amino acid (except for methionine and tryptophan) is encoded by a number of codons, the sequence of the nucleic acid molecule may be changed without changing the encoded amino acid. Codon optimization is when one or more codons are altered at the nucleic acid level such that the amino acids are not changed but expression in a particular host organism is increased. Those having ordinary skill in the art will recognize that codon tables and other references providing preference information for a wide range of organisms are available in the art (see, e.g., Zhang et al. (1991) Gene 105:61-72; Murray et al. (1989) Nucl. Acids Res. 17:477-508). Methodology for optimizing a nucleotide sequence for expression is provided, for example, in U.S. Pat. No. 6,015,891. Programs for codon optimization are available in the art (e.g., OPTIMIZER at genomes.urv.es/OPTIMIZER; OptimumGene.TM. from GenScript at: www.genscript.com/codon_opt.html).

The eukaryotic cell is preferably a Chinese hamster ovary (CHO) cell and accordingly the codon-optimization is preferably an optimization for the expression in CHO cells. CHO cells are of particular commercial interest since they are the most commonly used mammalian hosts for the industrial production of recombinant protein therapeutics.

Further details on suitable eukaryotic cells, including plant and animal cells will be provided herein below.

In Example 3 a Cps1 encoding nucleotide sequence is described being codon-optimized for the expression in plants (in particular rice).

The present invention relates in a second aspect to a vector encoding the nucleic acid molecule of the first aspect.

The definitions and preferred embodiments as described herein above apply *mutatis mutandis* to the second aspect, if applicable.

A vector according to this invention is generally and preferably capable of directing the replication, and/or the expression of the nucleic acid molecule of the invention and/or the expression of the polypeptide encoded thereby.

Preferably, the vector is a plasmid, cosmid, virus, bacteriophage or another vector used conventionally e.g. in genetic engineering.

Exemplary plasmids and vectors are listed, for example, in Studier and coworkers (Studier, W.F.; Rosenberg A.H.; Dunn J.J.; Dubendroff J.W., 1990, Use of the T7 RNA polymerase to direct expression of cloned genes, Methods Enzymol. 185, 61-89) or the brochures supplied by the companies Novagen, Promega, New England Biolabs, Clontech and Gibco BRL. Other preferred plasmids and vectors can be found in: Glover, D.M., 1985, DNA cloning: a practical approach, Vol. I-III, IRL Press Ltd., Oxford; Rodriguez, R.L. and Denhardt, D.T. (eds), 1988, Vectors: a survey of molecular cloning vectors and their uses, 179-204, Butterworth, Stoneham; Goedeel, D.V., 1990, Systems for heterologous gene expression, Methods Enzymol. 185, 3-7; Sambrook, J.; Russell, D. W., 2001, Molecular cloning: a laboratory manual, 3rd ed., Cold Spring Harbor Laboratory Press, New York.

Particularly preferred vectors are vectors that can be used for CRISPR genome editing, in particular vectors only expressing the nucleic acid molecule of the invention encoding the RNA-guided DNA endonuclease or vectors expressing both, the nucleic acid molecule of the invention encoding the RNA-guided DNA endonuclease and the guide RNA (so called "all-in one vectors"). In the former case a second vector is to be employed for the expression of the guide RNA. CRISPR genome editing vectors are commercially available, for example, from OriGene, Vector Builder or ThermoFisher.

The nucleic acid molecule of the present invention referred to above may also be inserted into vectors such that a translational fusion with another nucleic acid molecule is generated. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The products arising therefrom are termed fusion proteins and will be described further below. The other nucleic acid molecules may encode a protein which may e.g. increase the solubility and/or facilitate the purification of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see J.F. Sambrook and D.W. Russell, ed., Cold Spring Harbor Laboratory Press, 2001, ISBN-10 0-87969-577-3. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens et al., (2001), PNAS. 98 (4) 1471-1476) and optionally poly-A signals ensuring termination of transcription and stabilization of the transcript. Additional regulatory elements may include transcriptional as well as translational enhancers, and/or naturally-associated or heterologous promoter regions. The regulatory elements may be native to the endonuclease of the invention or heterologous regulatory elements. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

Additionally, baculoviral systems or systems based on Vaccinia Virus or Semliki Forest Virus can be used as vectors in eukaryotic expression systems for the nucleic acid molecules of the invention. Expression vectors derived from viruses such as retroviruses, vaccinia virus, adeno-associated virus, herpes viruses, or bovine papilloma virus, may be used for delivery of the nucleic acids or vector into targeted cell populations. Methods which are well known to those skilled in the art can be used to construct recombinant viral vectors; see, for example, the techniques described in Sambrook and D.W. Russell, ed., Cold Spring Harbor Laboratory Press, 2001.

Examples for regulatory elements permitting expression in eukaryotic host cells are promoters, including the promoters as described herein above. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the nucleic acid.

The co-transfection with a selectable marker such as kanamycin or ampicillin resistance genes for culturing in *E. coli* and other bacteria allows the identification and isolation of the transfected cells. Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS). Using such markers, the cells are grown in selective medium and the cells with the highest resistance are selected.

However, the nucleic acid molecules of the invention as described herein above may also be designed for direct introduction or for introduction via liposomes, phage vectors or viral vectors (e.g. adenoviral or retroviral) into the cell.

The present invention relates in a third aspect to a host cell comprising the nucleic acid molecule of the first aspect or being transformed, transduced or transfected with the vector of the second aspect.

The definitions and preferred embodiments as described herein above apply *mutatis mutandis* to the third aspect, if applicable.

Large amounts of the RNA-guided DNA endonuclease may be produced by said host cell, wherein the isolated nucleotide sequence encoding the RNA-guided DNA endonuclease is inserted into an appropriate vector or expression vector before insertion into the host. The vector or expression vector is introduced into an appropriate host cell, which preferably can be grown in large quantities, and the RNA-guided DNA endonuclease is purified from the host cells or the culture media.

The host cells may also be used to supply the RNA-guided DNA endonuclease of the invention without requiring purification of the RNA-guided DNA endonuclease (see Yuan, Y.; Wang, S.; Song, Z.; and Gao, R., Immobilization of an L-aminoacylase-producing strain of Aspergillus oryzae into gelatin pellets and its application in the resolution of D,L-methionine, Biotechnol Appl. Biochem. (2002). 35:107-113). The RNA-guided DNA endonuclease of the invention may be secreted by host cells. Those skilled in the field of molecular biology will understand that any of a wide variety of expression systems may be used to provide the RNA-guided DNA endonuclease. The precise host cell used is not critical to the invention, so long as the host cells produce the RNA-guided DNA endonuclease when grown under suitable growth conditions.

Host cells into which vectors containing the nucleic acid molecule of the invention can be cloned are used for replicating and isolating a sufficient quantity of the recombinant enzyme. The methods used for this purpose are well known to the skilled person (Sambrook and D.W. Russell, ed., Cold Spring Harbor Laboratory Press, 2001).

The expression of the RNA-guided DNA endonuclease may not only be used to produce the RNA-guided DNA endonuclease in a host cell, but its expression may also be used to edit the genome of the host cell. In such a case the host cell also comprises a guide RNA. Vectors that can be used for CRISPR genome editing have been discussed herein above.

In accordance with a preferred aspect of the third aspect of the invention the host cell is a eukaryotic cell or a prokaryotic cell and is preferably a plant cell or an animal cell.

The host cell can be a eukaryotic cell that does not naturally include a gene encoding Cps1 or Cps2, and can be, for example, be the cell of a fungus, algae, plant, or animal, wherein the animal can be an avian, reptile, amphibian, fish, cephalopod, crustacean, insect, arachnid, marsupial, or mammalian. The gene encoding Cps1 or Cps2 that is non-native with respect to the host cell can be operably linked to a regulatory element, such as a promoter. The promoter can be native to the host organism or can be a promoter of another species. A construct for expressing Cps1 or Cps2 in a heterologous host cell, such as a eukaryotic cell, can optionally further include a transcriptional terminator. The gene encoding Cps1 or Cps2 can optionally be codon optimized for the host species, can optionally include one or more introns, and can optionally include one or more peptide tag sequences, one or more nuclear localisation sequences (NLSs) and/or one or more linkers or engineered cleavage sites (e.g. a 2a sequence). In various embodiments a host cell can include any of the engineered Cps1 or Cps2 CRISPR systems disclosed above, where the nucleic acid sequence encoding the effector is present in the cell prior to introduction of a guide RNA. In other embodiments, the cell that is engineered to include a gene for expressing Cps1 or Cps2 polypeptide can further include a polynucleotide encoding a guide RNA (e.g., a guide RNA) that is operably linked to a regulatory element.

The cell or organism can be a prokaryotic cell that does not naturally include a gene encoding Cps1 or Cps2. Suitable prokaryotic host cells comprise e.g. bacteria of the species Escherichia, such as strains derived from *E. coli* BL21 (e.g. BL21(DE3), BL21(DE3)PlysS, BL21(DE3)RIL, BL21(DE3)PRARE, BL21 codon plus, BL21(DE3) codon plus), Rosetta®, XL1 Blue, NM522, JM101, JM109, JM105, RR1, DH5α, TOP 10, HB101 or MM294. Further suitable bacterial host cells are, but not limited to, *Streptomyces, Pseudomonas,* such as *Pseudomonas putida, Corynebacterium, such as C. glutamicum, Lactobacillus, such as L. salivarius* or *Bacillus* such as *Bacillus subtilis.*

In general, a eukaryotic host cell is preferred over a prokaryotic host cell.

The eukaryotic cell can be a yeast, fungus, amoebae, insect, vertebrate (e.g. mammalian) or plant cells.

Yeasts cells can be, for example, from *Saccharomyces cerevisiae, Ogataea angusta, Kluyveromyces sp. such as K. marxianus* or *K. lactis* or *Pichia sp.* such as *P. pastoris,* insect cells such as Drosophila S2 or Spodoptera Sf9 cells, plant cells, or fungi *cells, preferably of the* family *Trichocomaceae,* more preferably of the genus *Aspergillus, Penicillium* or *Trichoderma, or of the family Ustilaginaceae,* preferably *Ustilago sp..*

Plant host cells that may be used include monocots and dicots (i.e., monocotyledonous and dicotyledonous, respectively), preferably crop plant cells and tobacco cells. Crop plants include cereals (e.g. maize, wheat, rice), legumes (e.g. soybean, pea, and alfalfa), fruit and vegetables (e.g. lettuce, tomato, and potato). Examples of plant species of interest include, but are not limited to, corn (Zea mays), Brassica sp. (e.g., B. napus, B. rapa, B. juncea), particularly those Brassica species useful as sources of seed oil, alfalfa (Medicago sativa), rice (Oryza sativa), rye (Secale cereale), sorghum (Sorghum bicolor, Sorghum vulgare), camelina (Camelina sativa), millet (e.g., pearl millet (Pennisetum glaucum), proso millet (Panicum miliaceum), foxtail millet (Setaria italica), finger millet (Eleusine coracana)), sunflower (Helianthus annuus), quinoa (Chenopodium quinoa), chicory (Cichorium intybus), lettuce (Lactuca sativa), safflower (Carthamus tinctorius), wheat (Triticum aestivum), soybean (Glycine max), tobacco (Nicotiana tabacum), potato (Solanum tuberosum), peanuts (Arachis hypogaea), cotton (Gossypium barbadense, Gossypium hirsutum), sweet potato (Ipomoea batatus), cassava (Manihot esculenta), coffee (Coffea spp.), coconut (Cocos nucifera), pineapple (Ananas comosus), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oil palm (Elaeis guineensis), poplar (Populus spp.), eucalyptus (Eucalyptus spp.), oats (Avena sativa), barley (Hordeum vulgare), vegetables, ornamentals, and conifers.

Mammalian host cells that could be used include human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO) cells.

The present invention relates in a fourth aspect to a plant, seed or a part of a plant, said part of a plant no being a single plant cell, or an animal comprising the nucleic acid molecule of the first aspect or being transformed, transduced or transfected with the vector of the second aspect.

The definitions and preferred embodiments as described herein above apply *mutatis mutandis* to the fourth aspect, if applicable.

The plant or a part thereof may be a monocotyledonous plant or part thereof or a dicotyledonous plant or part thereof. The dicotyledonous plant is preferably a tobacco plant or part thereof. Likewise the seed may be a monocotyledonous or dicotyledonous plant seed and is preferably tobacco plant seed. Preferred examples of crop plants have been described herein above. Non-limiting examples of a part of a plant is a leave, stem, or root.

The animal is preferably a mammal and most preferably a non-human mammal. The mammal can be, for example, a mouse, rat, hamster, cat, dog, horse, swine, cattle, monkey, ape, etc.

By the expression of the nucleic acid molecule of the first aspect in a plant, seed or a part of a plant or an animal along with a guide RNA the genome of the host may be edited. The genome may be edited, for example, in order to introduce a targeted gene mutation, for gene therapy, for a creating chromosome rearrangement, for studying gene function, for the production of a transgenic organism, for endogenous gene labeling or for targeted transgene addition.

The present invention relates in a fifth aspect to a method of producing an RNA-guided DNA endonuclease comprising culturing the host cell of the third aspect and isolating the RNA-guided DNA endonuclease produced.

The definitions and preferred embodiments as described herein above apply *mutatis mutandis* to the fifth aspect, if applicable.

Suitable conditions for culturing a prokaryotic or eukaryotic host are well known to the person skilled in the art. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be overexpressed.

In general, *Aspergillus sp.* may be grown on Sabouraud dextrose agar, or potato dextrose agar at about to 10°C to about 40°C, and preferably at about 25°C. Suitable conditions for yeast cultures are known, for example from Guthrie and Fink, "Guide to Yeast Genetics and Molecular Cell Biology" (2002); Academic Press Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere. Suitable expression protocols for eukaryotic cells are well known to the skilled person and can be retrieved e.g. from Sambrook, 2001.

Methods for the isolation of the produced RNA-guided DNA endonuclease are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

The step of protein isolation is preferably a step of protein purification. Protein purification in accordance with the invention specifies a process or a series of processes intended to further isolate the polypeptide of the invention from a complex mixture, preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is useful for scientific purposes and the detection limits for protein are very low and nanogram amounts of protein are sufficient for their analysis. Proteins may also be purified by polarity/hydrophobicity via high performance liquid chromatography or reversed-phase chromatography. Thus, methods for protein purification are well known to the skilled person.

The present invention relates in a sixth aspect to an RNA-guided DNA endonuclease encoded by the nucleic acid molecule of the first aspect.

The definitions and preferred embodiments as described herein above apply *mutatis mutandis* to the sixth aspect, if applicable.

The amino acid sequences of SEQ ID NOs 1 and 3 are particularly preferred examples of the RNA-guided DNA endonuclease of the invention. Most preferred is an RNA-guided DNA endonuclease comprising or consisting of the amino acid sequence of SEQ ID NO: 1.

The RNA-guided DNA endonuclease of the sixth aspect of the invention may also be a fusion protein, wherein the amino acid sequence of the RNA-guided DNA endonuclease is fused to a fusion partner. The fusion may be a direct fusion or a fusion via a linker. The linker is preferably a peptide, such as a GS-linker.

The fusion partner can be located at the N-terminus, the C-terminus, at both termini, or in an internal location of the RNA-guided DNA endonuclease polypeptide, preferably at the N- or C-terminus.

The fusion partner is preferably a nuclear localization signal (NLS), a cell-penetrating domain, a plastid targeting signal, a mitochondrial targeting signal peptide, a signal peptide targeting both plastids and mitochondria, a marker domain, a tag (such as a purification tag), a DNA modifying enzyme or a transactivation domain.

DNA modifying enzymes may modify the DNA by phosphorylation, dephosphorylation of blunting DNA, wherein blunting refers the digestion single-stranded overhangs. Non-limiting examples of dephosphorylation enzymes are the Shrimp Alkaline Phosphatase (rSAP), Quick CIP Phosphatase and Antarctic Phosphatase. Non-limiting examples of phosphorylation enzymes are polynucleotide kinases, such as T4 PNK. Non-limiting examples of blunting enzymes are the DNA Polymerase I Large (Klenow) Fragment, T4 DNA Polymerase or Mung Bean Nuclease.

Transactivation domains (or trans-activating domains (TADs)) are transcription factor scaffold domains which contain binding sites for other proteins such as transcription co-regulators. Non-limiting examples are nine-amino-acid transactivation domains (9aaTADs) and Glutamine (Q)-rich TADs.

In general, an NLS comprises a stretch of basic amino acids. Nuclear localization signals are known in the art. The NLS can be at the N- terminus, the C-terminus or both the RNA-guided DNA endonuclease polypeptide according to the invention. For instance, the RNA-guided DNA endonuclease polypeptide according to the invention may comprise about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g., zero or at least one or more NLS at the amino-terminus and zero or at one or more NLS at the carboxy-terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C- terminus. The RNA-guided DNA endonuclease polypeptide sequence and the NLS may in some embodiments be fused with a linker between 1 to about 20 amino acids in length.

Non-limiting examples of NLSs include an NLS sequence derived from: the NLS of the SV40 virus large T-antigen; the NLS from nucleoplasmin (e.g., the nucleoplasmin bipartite NLS); the c-myc NLS; the hRNPAl M9 NLS; the IBB domain from importin-alpha; the NLS sequences of the myoma T protein, the p53 protein; the c-abl IV protein, or influenza virus NS 1 ; the NLS of the Hepatitis virus delta antigen, the Mxl protein; the poly(ADP-ribose) polymerase; and the steroid hormone receptors (human) glucocorticoid. In general, the one or more NLSs are of sufficient strength to drive accumulation of the RNA-guided DNA endonuclease polypeptide according to the invention in a detectable amount in the nucleus of a eukaryotic cell.

Plastid, mitochondrial, and dual-targeting signal peptide localization signals are also known in the art (see, e.g., Nassoury and Morse (2005) Biochim Biophys Acta 1743:5-19; Kunze and Berger (2015) Front Physiol 6:259; Herrmann and Neupert (2003) IUBMB Life 55:219-225; Soil (2002) Curr Opin Plant Biol 5:529-535; Carrie and Small (2013) Biochim Biophys Acta 1833:253-259; Carrie et al. (2009) FEBS J 276: 1187-1195; Silva-Filho (2003) Curr Opin Plant Biol 6:589-595; Peeters and Small (2001) Biochim Biophys Acta 1541:54-63; Murcha et al. (2014) Exp Bot 65:6301-6335; Mackenzie (2005) Trends Cell Biol 15:548-554; Glaser et al. (1998) Plant Mol Biol 38:311-338).

Non-limiting examples of marker domains include fluorescent proteins, purification tags, and epitope tags. In certain embodiments, the marker domain can be a fluorescent protein. Non-limiting examples of suitable fluorescent proteins include green fluorescent proteins (e.g., GFP, GFP-2, tagGFP, turboGFP, EGFP, Emerald, Azami Green, Monomeric Azami Green, CopGFP, AceGFP, ZsGreenl), yellow fluorescent proteins (e.g. YFP, EYFP, Citrine, Venus, YPet, PhiYFP, ZsYellowl), blue fluorescent proteins (e.g. EBFP, EBFP2, Azurite, mKalamal, GFPuv, Sapphire, T-sapphire), cyan fluorescent proteins (e.g. ECFP, Cerulean, CyPet, AmCyanl, Midoriishi-Cyan), red fluorescent proteins (mKate, mKate2, mPlum, DsRed monomer, mCherry, mRFPI, DsRed- Express, DsRed2, DsRed-Monomer, HcRed-Tandem, HcRedl, AsRed2, eqFP611, mRasberry, mStrawberry, Jred), and orange fluorescent proteins (mOrange, mKO, Kusabira-Orange).

A tag is short amino acid sequence that allows the identification of the RNA-guided DNA endonuclease polypeptide according to the invention in a mixture of polypeptides. Hence, the tag is preferably a purification tag. Non-limiting examples of a purification tag are a His-tag (e.g. His-6-tag). a GST-tag, DHFR-tag and a CBP-tag, A review of known purification tags can be found in Kimple et al. (2015), Curr Protoc Protein Sci. 2013; 73: Unit-9.9.

The present invention relates in a seventh aspect to a composition comprising the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof.

The definitions and preferred embodiments as described herein above apply *mutatis mutandis* to the seventh aspect, if applicable.

The term "composition" as used herein refers to a composition comprising at least one of the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof which are also collectively referred in the following as compounds.

In accordance with a preferred embodiment of the seventh aspect the composition is a pharmaceutical composition or a diagnostic composition.

In accordance with the present invention, the term "pharmaceutical composition" relates to a composition for administration to a patient, preferably a human patient. The pharmaceutical composition of the invention comprises at least one of the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by conventional methods. These pharmaceutical compositions may be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g of the active compound per day. However, a more preferred dosage might be in the range of 0.01 mg to 100 mg, even more preferably 0.01 mg to 50 mg and most preferably 0.01 mg to 10 mg per day. The length of treatment needed to observe changes and the interval following treatment for responses to occur vary depending on the desired effect. The particular amounts may be determined by conventional tests which are well known to the person skilled in the art.

The pharmaceutical composition may be used, for example, to treat or prevent a pathogenic disease, such as a viral or bacterial disease. For instance, the RNA-guided DNA endonuclease of the sixth aspect may be used together with gRNA targeting the genome of the pathogen thereby modifying the genome of the pathogen, such that the disease being caused by the pathogen is prevented or treated.

The pharmaceutical composition may also be used, for example, to treat or prevent a microbiome imbalance. An imbalance in the microbiome can occur, for example, because of an overuse of antibiotics, which may cause an overgrowth of pathogenic bacteria and yeast.

A "diagnostic composition" relates to composition which is suitable to detect a disease in subject, both infectious and non-infectious disease. The diagnostic composition may in particular comprise a marker portion as described herein above in connection with the fusion protein of the invention being attached to ssDNA strands, so that when RNA-guided DNA endonuclease polypeptide according to the invention cuts the ssDNA, it activates the reporter, causing it to fluoresce or change color, thus enabling visual detection of the specific disease nucleic marker. The diagnostic composition may be applied to a body fluid sample, such as a blood, urine, or saliva.

The present invention relates in a eighth aspect to the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof for use in the treatment of a disease in a subject or a plant by modifying a nucleotide sequence at a target site in the genome of the subject or plant.

Also described is method of treating or preventing a disease in a subject or a plant comprising modifying a nucleotide sequence at a target site in the genome of the subject or plant by the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof.

The definitions and preferred embodiments as described herein above apply *mutatis mutandis* to the eighth aspect, if applicable.

The modification of a nucleotide sequence at a target site in the genome of the subject or plant is in accordance with the invention a genome editing by the CRISPR technology and in particular by the novel RNA-guided DNA endonucleases as provided herewith which is to be used in combination with a proper gRNA and optionally a repair substrate as described herein below in order to determine the target side of the genome modification.

Genome editing (also known as genome engineering) is a type of genetic engineering in which a target site, preferably a gene of interest is inserted, deleted, modified or replaced in the genome of the cell. The target site, preferably the gene of interest can be in the genome but may also be in the mitochondrial DNA (animal cells) or chloroplast DNA (plant cells). Genome editing may result in a loss-of-function mutation or a gain-of-function mutation in the genome of the cell. A loss-of-function mutation (also called inactivating mutation) results in the gene of interest having less or no function (being partially or wholly inactivated). When the allele has a complete loss of function (wholly inactivated) this is also called herein a (gene) knock-out. A gene knock-out may be achieved by inserting, deleting, modifying or replacing one or more nucleotides of a gene. A gain-of-function mutation (also called activating mutation) may change the gene of interest such that its effect becomes stronger (enhanced activation) or even is superseded by a different (e.g. abnormal) function. A gain-of-function mutation may also introduce a new function or effect into a cell which the cell did not have before. In this context the new gene may be added to the genome of the cell (insertion) or may replace a gene within the genome. A gain-of-function mutation introducing such a new function or effect is also called gene knock-in. Genome editing may also result in the up- or down-regulation of one or more genes. By targeting DNA sites which are responsible for the regulation of the expression of a gene (e.g. a promoter region or a gene encoding transcription factor) the expression of genes can be up- or down-regulated by CRISPR technology. Further details on the mode of action of the CRISPR technology will be provided herein below.

Since its discovery, the CRISPR technology has been increasingly applied to therapeutic genome editing. Employment of several viral and non-viral vectors has enabled efficient delivery of the CRISPR system to target cells or tissues. In addition, the CRISPR system is able to modulate the target gene's expression in various ways, such as mutagenesis, gene integration, epigenome regulation, chromosomal rearrangement, base editing and mRNA editing (for review Le and Kim (2019), Hum Genet.;138(6):563-590).

The modification of a nucleotide sequence at a target site in the genome of the subject is preferably a gene therapy. Gene therapy is based on the principle of the genetic manipulation of nucleotide sequence at a target site for treating and preventing a disease, in particular a human disease.

In clinical trials of the CRISPR technology, scientists are using the CRISPR technology to combat cancer and blood disorders in humans. In these trials, some cells are removed from the subject to be treated, the DNA is genome-edited and then the genome edited cells are put back into the subject, said cells now being armed to fight the disease to be treated.

The present invention relates in a ninth aspect to a method of modifying a nucleotide sequence at a target site in the genome of a cell comprising introducing into said cell (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

Accordingly, the present invention also relates to a composition (e.g. a pharmaceutical or a diagnostic composition) comprising (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

The definitions and preferred embodiments as described herein above apply *mutatis mutandis* to the ninth aspect, if applicable.

The DNA-targeting RNA comprises a first segment comprising a nucleotide sequence that is complementary to a sequence in the target DNA and a second segment that interacts with the RNA-guided DNA endonuclease. As discussed herein above, the nucleotide sequence that is complementary to a sequence in the target DNA defines the target specificity of the RNA-guided DNA endonuclease. As also discussed herein above, the DNA-targeting RNA binds to the RNA-guided DNA endonuclease, whereby a complex is formed. The second segment interacts with the RNA-guided DNA endonuclease and is responsible for the formation of the complex. The second segment that interacts with the RNA-guided DNA endonuclease of the sixth aspect preferably comprises or consists of SEQ ID NO: 37 and more preferably of SEQ ID NO: 38 or 39. SEQ ID NO: 37 is a consensus sequence of the second segment of Type V Class 2 CRISPR nucleases. In Type V Class 2 CRISPR nucleases the second segment is also known as 5' handle. SEQ ID NO: 38 or 39 are the second segment of Cps1 and Cps2, respectively. SEQ ID NO: 38 and 39 are part of the stem loop sequences of SEQ ID NOS 28 and 29, respectively. Hence, the sequence comprising SEQ ID NO: 38 or 39 preferably comprises or consists of SEQ ID NO: 28 or 29, respectively.

The RNA-guided DNA endonuclease comprises as first segment being an RNA-binding portion that interacts with the DNA-targeting RNA and as a second segment being an activity portion that exhibits site-directed enzymatic activity. The first segment interacts with the DNA-targeting RNA and is responsible for the formation of the discussed complex. The second segment harbours the endonuclease domain, which preferably comprises a RuvC domain as described herein above (in particular a RuvC domain of SEQ ID NOs 12 to 14 or of SEQ ID NOs 15 to 17).

As also discussed herein above, the DNA-targeting RNA is the guide RNA. The guide RNA may either be directly introduced into the cells or as a DNA polynucleotide encoding the DNA-targeting RNA. In the latter case, the DNA encoding the guide RNA is generally operably linked to one or more promoter sequences for expression of the guide RNA. For example, the RNA coding sequence can be operably linked to a promoter sequence that is recognized by RNA polymerase III (Pol III) or RNA polymerase II (Pol II). The DNA polynucleotide encoding the DNA-targeting RNA is preferably a vector. Many single gRNA empty vectors (with and without the CRISPR endonuclease) are available in the art. Also several empty multiplex gRNA vectors are available that can be used to express multiple gRNAs from a single plasmid (with or without the expression of the CRISPR endonuclease). The DNA polynucleotide encodes the DNA-targeting RNA in expressible form.

Likewise, the RNA-guided DNA endonuclease may either be directly introduced into the cells or as a nucleic acid molecule encoding the RNA-guided DNA endonuclease, the latter being preferably a vector of the second aspect. The DNA polynucleotide encodes the RNA-guided DNA endonuclease in expressible form.

As is discussed in greater detail herein above, the RNA-guided DNA endonuclease and the DNA-targeting RNA may also be encoded by the same DNA polynucleotide, such as an all-in one CRISPR-cas vector.

The term "in expressible form" means that the one or more DNA polynucleotides encoding the RNA-guided DNA endonuclease and the DNA-targeting RNA are in a form that ensures that the DNA-targeting RNA is transcribed and that the RNA-guided DNA endonuclease is transcribed and translated into the active enzyme in the cells.

In accordance with a preferred embodiment of the ninth aspect of the invention in case the RNA-guided DNA endonuclease and the DNA-targeting RNA are directly introduced into the cell they are introduced in the form of a ribonucleoprotein complex (RNP).

RNPs are assembled *in vitro* and can be delivered to the cell by methods known in the art, for example, electroporation or lipofection. RNPs are capable to cleave the target site with comparable efficacy as nucleic acid-based (e.g. vector-based) RNA-guided DNA endonucleases (Kim et al. (2014), Genome Research 24(6):1012-1019).

Means for introducing proteins (or peptides) or RNPs into living cells are known in the art and comprise but are not limited to microinjection, electroporation, lipofection (using liposomes), nanoparticle-based delivery, and protein transduction. Any one of these methods may be used.

A liposome used for lipofection is a small vesicle, composed of the same material as a cell membrane (i.e., normally a lipid bilayer e.g. made of phospholipids), which can be filled with one or more protein(s) (e.g. Torchilin VP. (2006), Adv Drug Deliv Rev., 58(14):1532-55). To deliver a protein or RNP into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Feigner et al. (1994). J. Biol. Chem. 269, 2550-2561). Nanoparticle-based delivery of Cas9 ribonucleoprotein and donor DNA for the induction of homology-directed DNA repair is, for example, described in Lee et al. (2017), Nature Biomedical Engineering, 1:889-90.

Protein transduction specifies the internalisation of proteins into the cell from the external environment (Ford et al (2001), Gene Therapy, 8:1-4). This method relies on the inherent property of a small number of proteins and peptides (preferably 10 to 16 amino acids long) to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability, protein (or peptides or nucleic acid sequences) can be introduced into the living cell.

The RNA-guided DNA endonuclease may be introduced into the cells as an active enzyme or as a proenzyme. In the latter case the RNA-guided DNA endonuclease is biochemically changed within the cells (for example by a hydrolysis reaction revealing the active site, or changing the configuration to reveal the active site), so that the proenzyme becomes an active enzyme.

Means and methods for the introduction of nucleic acid molecule(s) and DNA-targeting RNA into cells are likewise known in the art and these methods encompass transducing or transfecting cells.

Transduction is the process by which foreign DNA is introduced into a cell by a virus or viral vector. Transduction is a common tool used by molecular biologists to stably introduce a foreign gene into a host cell's genome. Generally, a plasmid is constructed in which the genes to be transferred are flanked by viral sequences that are used by viral proteins to recognize and package the viral genome into viral particles. This plasmid is inserted (usually by transfection) into a producer cell together with other plasmids (DNA constructs) that carry the viral genes required for formation of infectious virions. In these producer cells, the viral proteins expressed by these packaging constructs bind the sequences on the DNA/RNA (depending on the type of viral vector) to be transferred and insert it into viral particles. For safety, none of the plasmids used contains all the sequences required for virus formation, so that simultaneous transfection of multiple plasmids is required to get infectious virions. Moreover, only the plasmid carrying the sequences to be transferred contains signals that allow the genetic materials to be packaged in virions, so that none of the genes encoding viral proteins are packaged. Viruses collected from these cells are then applied to the cells to be altered. The initial stages of these infections mimic infection with natural viruses and lead to expression of the genes transferred and (in the case of lentivirus/retrovirus vectors) insertion of the DNA to be transferred into the cellular genome. However, since the transferred genetic material does not encode any of the viral genes, these infections do not generate new viruses (the viruses are "replication-deficient"). In the present case transduction may be used to generate cells that comprise the RNA-guided DNA endonuclease in their genome in expressible form.

Transfection is the process of deliberately introducing naked or purified nucleic acids or purified proteins or assembled ribonucleoprotein complexes into cells. Transfection is generally a non-viral based method.

Transfection may be a chemical-based transfection. Chemical-based transfection can be divided into several kinds: transfection using cyclodextrin, polymers, liposomes, or nanoparticles. One of the cheapest methods uses calcium phosphate. HEPES-buffered saline solution (HeBS) containing phosphate ions are combined with a calcium chloride solution containing the DNA to be transfected. When the two are combined, a fine precipitate of the positively charged calcium and the negatively charged phosphate will form, binding the DNA to be transfected on its surface. The suspension of the precipitate is then added to the cells to be transfected (usually a cell culture grown in a monolayer). By a process not entirely understood, the cells take up some of the precipitate, and with it, the DNA. This process has been a preferred method of identifying many oncogenes. Other methods use highly branched organic compounds, so-called dendrimers, to bind the DNA and transfer it into the cell. Another method is the use of cationic polymers such as DEAE-dextran or polyethylenimine (PEI). The negatively charged DNA binds to the polycation and the complex is taken up by the cell via endocytosis. Lipofection (or liposome transfection) is a technique used to inject genetic material into a cell by means of liposomes, which are vesicles that can easily merge with the cell membrane since they are both made of a phospholipid bilayer, as mentioned above. Lipofection generally uses a positively charged (cationic) lipid (cationic liposomes or mixtures) to form an aggregate with the negatively charged (anionic) genetic material. This transfection technology performs the same tasks in terms of transfer into cells as other biochemical procedures utilizing polymers, DEAE-dextran, calcium phosphate, and electroporation. The efficiency of lipofection can be improved by treating transfected cells with a mild heat shock. Fugene is a series of widely used proprietary non-liposomal transfection reagents capable of directly transfecting a wide variety of cells with high efficiency and low toxicity.

Transfection may also be a non-chemical method. Electroporation (gene electrotransfer) is a popular method, where transient increase in the permeability of cell membrane is achieved when the cells are exposed to short pulses of an intense electric field. Cell squeezing enables delivery of molecules into cells via cell membrane deformation. Sonoporation uses high-intensity ultrasound to induce pore formation in cell membranes. This pore formation is attributed mainly to the cavitation of gas bubbles interacting with nearby cell membranes since it is enhanced by the addition of ultrasound contrast agent, a source of cavitation nuclei. Optical transfection is a method where a tiny (^{∼}1 µm diameter) hole is transiently generated in the plasma membrane of a cell using a highly focused laser. Protoplast fusion is a technique in which transformed bacterial cells are treated with lysozyme in order to remove the cell wall. Following this, fusogenic agents (e.g., Sendai virus, PEG, electroporation) are used in order to fuse the protoplast carrying the gene of interest with the recipient target cell.

Finally, transfection may be a particle-based method. A direct approach to transfection is the gene gun, where the DNA is coupled to a nanoparticle of an inert solid (commonly gold), which is then "shot" (or particle bombardment) directly into the target cell's nucleus. Hence, the nucleic acid is delivered through membrane penetration at a high velocity, usually connected to microprojectiles. Magnetofection, or magnet-assisted transfection, is a transfection method that uses magnetic force to deliver DNA into target cells. Impalefection is carried out by impaling cells by elongated nanostructures and arrays of such nanostructures such as carbon nanofibers or silicon nanowires which have been functionalized with plasmid DNA.

The method of the ninth aspect of the invention relates to a method for editing (i.e. "mutating") with the RNA-guided DNA endonuclease of the invention a nucleotide sequence at a target site in the genome of a cell. This requires essentially three sequential preconditions: (1) Efficient delivery of the RNA-guided DNA endonuclease-encoding genes or the RNA-guided DNA endonuclease itself into the target cell; (2) efficient expression or presence of the CRISPR-components in the target cell (DNA-targeting RNA and the RNA-guided DNA endonuclease of the sixth aspect); and (3) targeting of the genomic site of interest by CRISPR ribonucleoprotein complexes and repair of the DNA by cell's own repair pathways. Step (3) is automatically carried out in the cell upon the expression of the CRISPR-components in the cell the genome of which is to be edited.

By genome editing a target site may be inserted, deleted, modified (including singe nucleotide polymorphisms (SNPs)) or replaced in the genome of the cell. The target site can be in the coding region of a gene, in an intron of a gene, in a control region of a gene, in a non-coding region between genes, etc. The gene can be a protein coding gene or an RNA coding gene. The gene can be any gene of interest.

In this connection genome editing uses the cell's own repair pathways, including the non-homologous end-joining (NHEJ) or homology directed recombination (HDR) pathway. Once the DNA is cut by the RNA-guided DNA endonuclease, the cell's own DNA repair machinery (NHEJ or HDR) adds or deletes pieces of genetic material, or makes changes to the DNA by replacing an existing segment with a customized DNA sequence. Hence, in the CRISPR-Cas system, the CRISPR nuclease makes a double-stranded break in DNA at a site determined by the short (^{∼}20 nucleotide) gRNA which break is then repaired within the cell by NHEJ or HDR. It is preferred that genome editing uses NHEJ. In a different embodiment, it is preferred that genome editing uses HDR.

NHEJ uses a variety of enzymes to directly join the DNA ends in a double-strand break. In contrast, in HDR, a homologous sequence is utilized as a template for the regeneration of missing DNA sequence at the break point. NHEJ is the canonical homology-independent pathway as it involves the alignment of only one to a few complementary bases at most for the re-ligation of two ends, whereas HDR uses longer stretches of sequence homology to repair DNA lesions.

The natural properties of these pathways form the very basis of RNA-guided DNA endonuclease-based genome editing. NHEJ is error-prone, and has been shown to cause mutations at the repair site. Thus, if one is able to create a double strand break (DSB) at a desired gene in multiple samples, it is very likely that mutations will be generated at that site in some of the treatments because of errors created by the NHEJ infidelity. On the other hand, the dependency of HDR on a homologous sequence to repair DSBs can be exploited by inserting a desired sequence within a sequence that is homologous to the flanking sequences of a DSB which, when used as a template by the HDR system, would lead to the creation of the desired change within the genomic region of interest. Despite the distinct mechanisms, the concept of the HDR based gene editing is in a way similar to that of homologous recombination based gene targeting. So based on these principles if one is able to create a DSB at a specific location within the genome, then the cell's own repair systems will help in creating the desired mutations.

The homologous sequence template for HDR is also referred to herein as "repair template".

Hence, by modifying a nucleotide sequence at a target site in the genome of a cell according to the ninth aspect of the invention a gene may be knocked-out (by introducing per-mature stop codon) or knocked-in (via the repair substrate). It is likewise possible to alter the expression of a gene by the method of the ninth aspect of the invention. For instance, the target site in the genome may be a promoter region changing the promoter region may increase or decrease the expression of the gene being controlled via the target promoter region.

Hence, in accordance with a preferred embodiment of the ninth aspect the method further comprises the introduction of a repair substrate into said cell.

The repair substrate may either be directly introduced into the cell or as a nucleic acid molecule, preferably a vector encoding the repair substrate in expressible from. The use of a repair substrate for correcting a mutant beta-glucuronidase in wheat embryo is illustrated in Example 3.

The designs and structures of repair templates being suitable for HDR are known in the art. HDR is error-free if the repair template is identical to the original DNA sequence at the double-strand break (DSB), or it can introduce very specific mutations into DNA. The three central steps of the HDR pathways are: (1) The 5'-ended DNA strand is resected at the break to create a 3' overhang. This will serve as both a substrate for proteins required for strand invasion and a primer for DNA repair synthesis. (2) The invasive strand can then displace one strand of the homologous DNA duplex and pair with the other. This results in the formation of the hybrid DNA, referred to as the displacement loop (D loop). (3) The recombination intermediates can then be resolved to complete the DNA repair process.

HDR templates used, for example, to introduce mutations or insert new nucleotides or nucleotide sequences into a gene require a certain amount of homology surrounding the target sequence that will be modified. Homology arms can be used that start at the CRISPR-induced DSB. In general, the insertion sites of the modification should be very close to the DSB, ideally less than 10 bp away, if possible. One important point to note is that the CRISPR enzymes may continue to cleave DNA once a DSB is introduced and repaired. As long as the gRNA target site/PAM site remain intact, the CRISPR nuclease will keep cutting and repairing the DNA. This repeated editing may be problematic if a very specific mutation or sequence is to be introduced into a gene of interest. To get around this, the repair template can be designed in such a way that it will ultimately block further CRISPR nuclease targeting after the initial DSB is repaired. Two common ways to block further editing are mutating the PAM sequence or the gRNA seed sequence. When designing a repair template, the size of the intended edit is to be taken into consideration. ssDNA templates (also referred to as ssODNs) are commonly used for smaller modifications. Small insertions/edits may require as little as 30-50 bases for each homology arm, and the best exact number may vary based on the gene of interest. 50-80 base homology arms are commonly used. For example, Richardson et al. (2016). Nat Biotechnol. 34(3):339-44) found that asymmetric homology arms (36 bases distal to the PAM and 91 bases proximal to the PAM) supported HDR efficiencies up to 60%. Due to difficulties that might be associated with creating ssODNs longer than 200 bases, it is preferred to use dsDNA plasmid repair templates for larger insertions such as fluorescent proteins or selection cassettes into a gene of interest. These templates can have homology arms of at least 800 bp. To increase the frequency of HDR edits based on plasmid repair templates, self-cleaving plasmids can be used that contain gRNA target sites flanking the template. When the CRISPR nuclease and the appropriate gRNA(s) are present, the template is liberated from the vector. To avoid plasmid cloning, it is possible to use PCR-generated long dsDNA templates. Moreover, Quadros et al. (2017) Genome Biol.17;18(1):92) developed Easi-CRISPR, a technique that allows making large mutations and to take advantage of the benefits of ssODNs. To create ssODNs longer than 200 bases, RNA encoding the repair template are *in vitro* transcribed and then reverse transcriptase is used to create the complementary ssDNA. Easi-CRISPR works well in mouse knock-in models, increasing editing efficiency from 1-10% with dsDNA to 25-50% with ssODNs. Although HDR efficiency varies across loci and experimental systems, ssODN templates generally provide the highest frequency of HDR edits.

In accordance with a preferred embodiment of the ninth aspect the cell is not the natural host of a gene encoding said RNA-guided DNA endonuclease.

As discussed herein above, the RNA-guided DNA endonucleases of SEQ ID NOs 1 and 3 were isolated from a metagenome library comprising bacteria that cannot be cultured. For this reason the exact nature of the bacteria from which SEQ ID NOs 1 and 3 were isolated is not known.

Hence, the cell that is not the natural host of SEQ ID NOs 1 and 3 may be any bacterial cell from a bacterial strain that can be cultured or any eukaryotic cell. Such manipulated cells do not exist in nature.

In accordance with another preferred embodiment of the ninth aspect the cell is a eukaryotic cell, preferably a plant cell or animal cell.

Eukaryotic cells, plant cells and animal cells as well as eukaryotes, plants and animals from which cells may be obtained including preferred examples thereof have been described herein above in connection with the third and fourth aspect of the invention.

These cells may likewise be used in connection with the ninth aspect of the invention.

In accordance with a more preferred embodiment of the ninth aspect the method further comprises culturing the plant cell or animal cell to produce a plant or animal under conditions in which the RNA-guided DNA endonuclease is expressed and cleaves the nucleotide sequence at the target site to produce a modified nucleotide sequence; and selecting a plant or animal comprising said modified nucleotide sequence.

In this connection the cell(s) into which the components of the CRISPR-Cas system are to be introduced has/have to be a totipotent stem cell or a germ line cell (oocyte and/or sperm) or a collection of stem cells being capable of developing into a complete plant or animal. Means and method for culturing such cell(s) in order to produce a plant or animal are known in the art (see for example, https://www.stembook.org/node/720).

Unless otherwise defined, all technical and scientific terms used herein have the same meaning as commonly understood by one of ordinary skill in the art to which this invention belongs. In case of conflict, the patent specification including definitions, will prevail.

The present invention relates in a tenth aspect to modified cells that have been produced by the method according to the ninth aspect of the invention for use in treatment of a disease in a subject.

The modified cells are preferably modified T lymphocytes and the disease to be treated is preferably cancer (Stadtmauer et al., Science 28 Feb 2020:Vol. 367, Issue 6481, eaba7365).

The cells to be modified by the method of the ninth aspect of the invention are preferably obtained from the subject to be treatment and then the modified cells are used in accordance with the tenth aspect of the invention.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

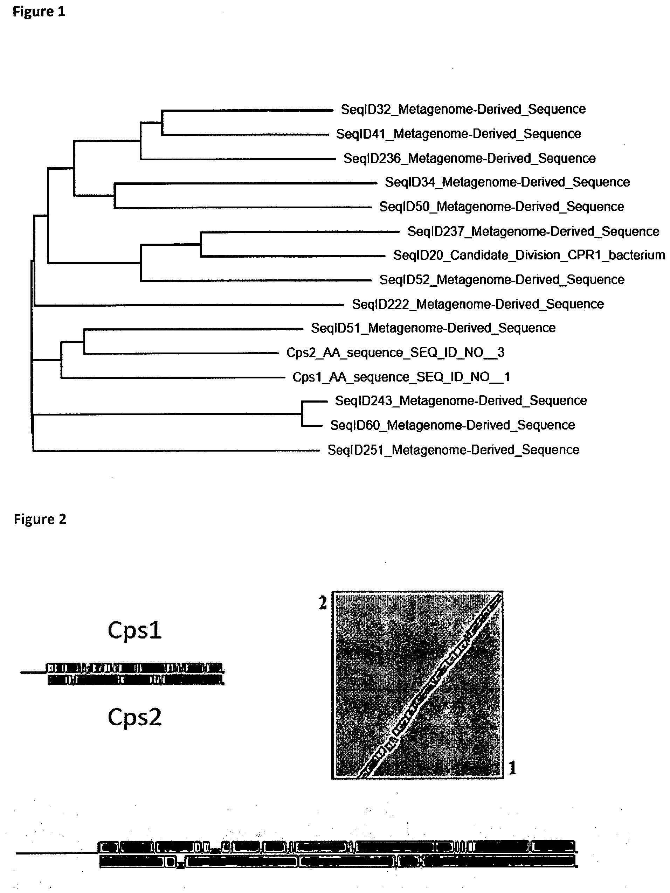

**Fig. 1** shows a centred phylogenetic tree comprising related class 2, type V nucleases identified in public sequence databases, with Cps1 and Cps2. All next neighbour sequences were disclosed in US 2019/0048357, the highest sequence identity on amino acid level being SeqID 51 with 42% for Cps1 and 48% for Cps2, respectively.
**Fig. 2** Comparison of the amino acid sequences of the RNA-guided DNA endonucleases of the invention Cps1 (SEQ ID NO: 1) and Cps2 (SEQ ID NO: 3)
**Fig 3****.** Stem Loop Direct Repeat Similarity between Cps1 (SEQ ID NO:28) and Cps2 (SEQ ID NO: 29) in comparison to other class 2 type V CRISPR nucleases (SEQ ID NOs 30 to 32)
**Fig 4****.** Comparison of RuvC Domains of Cps1 (SEQ ID NO: 12-14) and Cps2 (SEQ ID NO: 15-17) in comparison to other class 2 type V CRISPR nucleases (SEQ ID NOs 18-26)
**Fig. 5** Wheat embryo genome editing assay: diagram of experimental setup for the repair of an inactive, mutant β-glucuronidase (mutant GUS) gene *in vivo* in wheat cells mediated by nuclease activity of the Cps1 protein and a crRNA from co-delivered genes

The examples illustrate the invention.

### EXAMPLES

### Example 1 - Habitat selection and DNA preparation

An approach for the discovery of novel Cas proteins is to access metagenomics resources in terabase scale by Next Generation Sequencing of selected environmental DNA (e.g. 1 cm³ forest soil contains ^{∼} 2.5 x 10¹⁰ bp DNA or ^{∼}20 million genes) and computational identification of CRISPR-Cas systems. To this aim, metagenome habitats were selected from different locations in Germany. Aquatic, sediment and soil habitats were sampled and preprocessed before the DNA isolation. A staggered filtration process using a variety of filters of different pore sizes (0.1 - 20 µm) were used to enrich Candidata Phyla Radiation (CPR) species containing a nearly untouched space of novel sequences (Hug et al. (2016) Nature Microbiology 1, 16048).
DNA was extracted from 0.2 and 0.1 µm filters and isolated using the PowerWater DNA isolation Kit (Qiagen).

### Example 2 - Next Generation Sequencing and sequence evaluation

DNA libraries for Next Generation Sequencing were prepared using the TruSeq PCR free Library Prep Kit (Illumina) and the metagenomics libraries were sequenced using a HiSeq 2500 (Illumina) in Rapid Mode (2 x 250 Bp) generating an average output of 50 Gbp per sample.

The generated paired end metagenome sequence libraries were assembled, annotated and evaluated to identify potential CRISPR operons by the identification of CRISPR repeats. The contigs containing the identified CRISPR repeat-spacer units were used to analyze the surrounding regions for the presence of Cas proteins. Open reading frames in close proximity to the CRISPR repeats were analyzed for their pattern and sequences by comparing them to genome and protein databases to identify potential novel Cas proteins.

BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) supra. When utilizing BLAST, Gapped BLAST, PSI-BLAST, the default parameters of the respective programs (e.g., BLASTN for nucleotide sequences, BLASTX for proteins) can be used. See the website at www.ncbi.nlm.nih.gov. Alignment may also be performed manually by inspection.

The Cps1 sequence was discovered in a metagenomics sediment sample collected from a barrier lake (Germany). The contig was identified due to the presence of a CRISPR array containing six repeat spacer units. Analysis of the surrounding area revealed a long ORF in close proximity to the identified CRISPR array showing a slight sequence homology towards Cms1 like CRISPR nucleases described by Benson Hill Biosystems Inc. (WO 2019/030695).
The Cps2 sequence was discovered in a metagenomics sediment sample collected from a fish pond (Germany). The contig was identified due to the presence of a CRISPR array containing 48 repeat spacer units. As described above, analysis of the surrounding area revealed a long ORF showing a slight sequence homology towards Cms1 like CRISPR nucleases as described in WO 2019/030695.

### Translation into protein sequences

Using the "standard" translation table 1 (https://www.ncbi.nlm.nih.gov/Taxonomy/Utils/wprintgc.cgi) to translate the DNA sequence of Cps1 and Cps2 into an amino acid sequence it was observed that both identified protein sequences were interrupted by several stop codons. To further analyse the sequences, the species containing the identified DNA sequences were determined by sequence homology. To do so, the complete DNA contigs containing the Cps1 and Cps2 sequences were analysed for their sequence homology towards already existing genomic information by using various public DNA databases. Using this approach it could be shown that the Cps1 as well as the Cps2 containing contigs have slight sequence homologies towards organisms belonging to the group of Candidate division SR1 bacterium. Furthermore, it is known that organisms belonging to the Candidate division group are using a specific translation table (translation table 25: https://www.ncbi.nlm.nih.gov/Taxonomy/Utils/wprintgc.cgi).
Assuming that the Cps1 and Cps2 containing organisms are from the same group of microorganisms the described translation table 25 was used to translate the open reading frames of Cps1 and Cps2 generating an amino acid sequence without interruption by stop codons.

### Example 3 - GUS assay for functional validation of Cps1 nuclease activity

In this example, an inactive, mutant β-glucuronidase (mutant GUS) gene was repaired *in vivo* in wheat cells mediated by nuclease activity of the Cps1 protein and a crRNA from co-delivered genes.

### Materials and methods

### Preparation of wheat immature embryos

Immature embryos were collected from wheat cv. Fielder essentially according to the methods described by Ishida and Hiei (2011). WO2011/013764 and Ishida et al. (2015) Methods in Molecular Biology, vol. 1223. Immature seeds of 14 days after pollination were de-husked, surface-sterilized by 70% ethanol for 45 sec and then by 1% sodium hypochlorite with one drop per 50 ml of Tween20 for 10 min, and rinsed 4 times with sterile water. Immature embryos were isolated from the seeds under stereoscopic microscope and collected into WLS-liq medium (0.1 x LS major salts, 10 µM FeEDTA, 0.1 x LS minor salts, 0.1 x MS vitamins, 1% glucose, 0.05% MES, pH5.8). The embryos were washed with WLS-liq medium water, immersed in WLS-liq medium, and centrifuged at 4500 x g at 4 °C for 10 min. The embryo axes were removed from the embryos. The embryos were cultured on WLS medium (LS major salts, 100 µM FeEDTA, LS minor salts, MS vitamins, 0.5 mg/L 2.4-D, 2.5 mg/L Dicamba, 9% Maltose, 500 mg/L glutamine, 100 mg/L casein hydrolysate, 3.7 mM MgCl₂, 0.195% MES, 57 µM ascorbic acid, 5 µM AgNO₃, 5 g/L agarose, pH5.8) with the scutellum side up in the dark at 25°C for 1 day.

### Vector construction

The sequence of the active GUS gene is shown as SEQ ID NO: 5. The stretch from nucleotide No. 16 to nucleotide No. 205 is an intron from the catalase gene of castor bean. The sequence of the mutant GUS is shown as SEQ ID NO: 6. The mutant GUS gene was created by inserting 55 nucleotides of DNA from nucleotide No. 489 to nucleotide No. 583 of SEQ ID NO: 6 into the GUS gene at the location after nucleotide No. 488 of SEQ ID NO: 5. The first 15 nucleotides of the insert contained stop codons in all three reading frames, and the rest of 40 nucleotides were identical to the stretch of 40 nucleotides preceding the insertion site to generate direct repeats. Because of this insertion, the mutant GUS could not produce active GUS protein.
The sequences of SmCms1 gene, Cps1 gene, and SpCas9 gene are shown as SEQ ID NO: 7, SEQ ID NO: 8, and SEQ ID NO: 9, respectively. The SmCms1 gene was codon-optimized from the sequence reported by Begemann et al. (2017) bioRxiv doi: https://doi.org/10.1101/192799 for expression in wheat. The Cps1 gene was codon-optimized for expression in rice. The 9 amino acids at the N-termini coded by these genes are the nuclear localization signal (NLS) from SV40. The 18 amino acids at the C-termini coded by the SmCm1 gene and Cps1 gene are the NLS from virD2 protein.

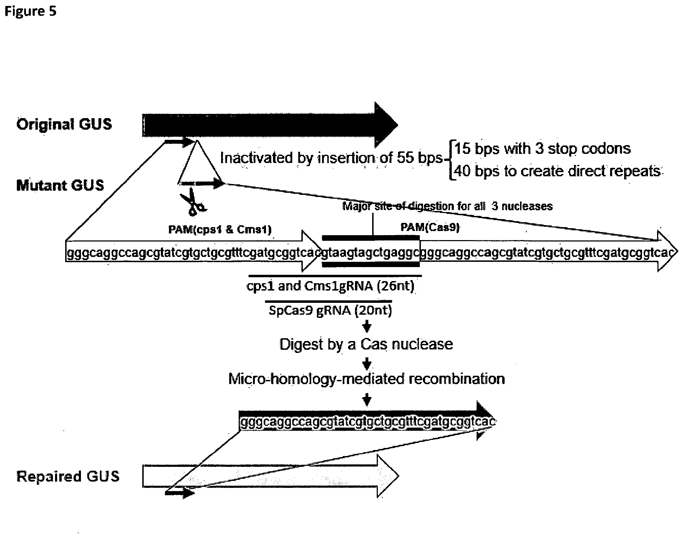

The GUS gene, the mutant GUS gene, the SmCms1 gene, the Cps1 gene, and the SpCas9 gene were inserted between the ubiquitin promoter carrying the intron and the terminators from the nos gene and the 35S transcript of the cauliflower mosaic virus on pUC18, respectively.
The sequences of the expression units of the sgRNA for the SpCas9 and of the crRNA for SmCms1 and Cps1 are shown as SEQ ID NO: 10 and SEQ ID NO: 11, respectively. Both of them include the promoter and terminator of wheat U6 polymerase III Shan et al. (2014). Nat Protoc. 9(10):2395-410.These sequences were designed so that the mutant GUS gene would be digested most likely at a site between the direct repeats (Figure 5). These sequences were cloned into pUC18. For Cas9 the PAM sequence AGG was used whereas the PAM sequence of SmCms1 and Cps1 was TTTC.

### DNA delivery by particle bombardment

DNA was delivered to the immature embryos essentially according to the procedures described by Barcelo and Lazzeri (1995). Humana Press Inc, 113-123. and Rasco-Gaunt et al. (2001). J. Exp. Bot. 52, 865-874. 0.5 ml of 70% ethanol was added to 15 mg of Bio-Rad 0.6 µm gold particles in 1.5 ml microtube. The mixture was sonicated for 90 sec, shaken for 15 min and centrifuged for 5 sec. The supernatant was discarded. The particles were suspended in 0.5 ml sterile water, vortexed, and centrifuged for 5 sec. The supernatant was discarded. This process of suspension and centrifugation was repeated 3 times. The particles were suspended in 0.375 ml of 50% glycerol to make the final concentration of 40 mg/ml, stored at 4 °C, and vortexed for 5 min before use.
20 µl of the gold suspension (0.8 mg of the particles), 20 µl of DNA solution, and 0.8 µl of TranslT®-2020 Transfection Reagent (TaKaRa) were mixed in a fresh tube in this order, while being vortexed frequently. The tube was vortexed for 3 min, kept without agitation for 1 min, and centrifuged briefly. The supernatant was discarded, and 56 µl of 70% ethanol was added to the pellet. The tube was vortexed and centrifuged briefly. The supernatant was discarded, and 56 µl of 100% ethanol was added to the pellet. The tube was vortexed and centrifuged briefly. The supernatant was discarded. The pellet was suspended in 34 µl of 100% ethanol by gentle tapping, and vortexed for 3 sec. The tube was sonicated for 30 sec. 5 µl of the gold suspension was applied to a shot of bombardment. The embryos cultured on the WLS medium were bombarded by Bio-Rad PDS 1000/He biolistic gun with a pressure of 650 psi according to the instruction by the manufacturer. The culture was placed onto the shelf at a distance of 5 cm from the stopping screen. 16 of the embryos were targeted by a shot of bombardment. After the bombardment, the culture was incubated in the dark at 30 °C for 1 day and then at 25 °C for 4 days

### Detection of GUS expression

The embryos were incubated in 0.1% Triton X-100 in 0.1 M phosphate buffer of pH 6.8 at 37 °C for 1 hr. The solution was removed, and the embryos were incubated in 1.0 mM 5-bromo-4-chloro-3-indolyl-β-D-glucuronide in 0.1 M phosphate buffer of pH 6.8 with 20% methanol at 37 °C for 1 day. Blue spots showing GUS expression were visually examined.

### Experiments and results

The gene repair strategy for mutant GUS is shown schematically in Fig. 5. The experiments and results are summarized in Table 1. In both experiments, strong GUS expression was detected in the positive control of Plot 5 whereas no expression was detected in the negative control of Plot 4. Therefore, it was confirmed that the mutant GUS gene was indeed inactive, and the experiments were well controlled.

On the other hand, GUS expression was detected from the mutant GUS in Plot 1, Plot 2, and Plot 3. The most plausible interpretation of the results is that the mutant GUS gene was digested by a combination of the Cps1 and the crRNA, the SmCms1 and the crRNA, or SpCas9 and sgRNA, and then repaired most likely by recombination mediated by the micro-homology between the direct repeats (McVey, 2008). Nature Protocols. Vol.9 No.10. Once the mutant GUS gene was digested at a site close to the direct repeats, other mechanisms, such as a combination of a certain length of deletion and non-homologous end joining, could also repair the mutant GUS. However, the mutant GUS could hardly be repaired without such digestion. These results confirm that Cps1 is a functional Type V CRISPR nuclease, does not require a tracrRNA and is able to introduce double strand breaks adjacent to a TTTC PAM motive.

**Table 1. GUS expression in wheat immature embryos after repair of mutant GUS**

| Plot | DNA molecules delivered | GUS expression |
|---|---|---|
| 1 | 30 pmol Cps1, 100 pmol crRNA, 500 pmol mutant GUS | + |
| 2 | 30 pmol SmCms1, 100 pmol crRNA, 500 pmol mutant GUS | + |
| 3 | 30 pmol SpCas9, 100 pmol sgRNA, 500 pmol mutant GUS | + |
| 4 | 500 pmol mutant GUS | - |
| 5 | 50 pmol GUS | +++ |

| | | |
|---|---|---|
| -: No blue spots +: A few blue spots +++: Many blue spots | | |

## Claims

1. A nucleic acid molecule encoding an RNA-guided DNA endonuclease, which is
(a) a nucleic acid molecule encoding the RNA-guided DNA endonuclease comprising or consisting of the amino acid sequence of SEQ ID NO: 1 or 3;
(b) a nucleic acid molecule comprising or consisting of the nucleotide sequence of SEQ ID NO: 2 or 4;
(c) a nucleic acid molecule encoding a RNA-guided DNA endonuclease the amino acid sequence of which is at least 70 % identical to the amino acid sequence of (a); preferably at least 80 % identical, more preferably at least 90 % identical, and most preferred at least 95% identical;
(d) a nucleic acid molecule comprising or consisting of a nucleotide sequence which is at least 70 % identical to the nucleotide sequence of (b), preferably at least 80 % identical, more preferably at least 90 % identical, and most preferred at least 95% identical;
(e) a nucleic acid molecule which is degenerate with respect to the nucleic acid molecule of (d); or
(f) a nucleic acid molecule corresponding to the nucleic acid molecule of any one of (a) to (d) wherein T is replaced by U.

2. The nucleic acid molecule of claim 1, wherein the nucleic acid molecule is operably linked to a promoter that is native or heterologous to the nucleic acid molecule.

3. The nucleic acid molecule of claim 1 or 2, wherein said nucleic acid molecule is codon-optimized for expression in a eukaryotic cell, preferably a plant cell or an animal cell.

4. A vector encoding the nucleic acid molecule of any one of claims 1 to 3.

5. A host cell comprising the nucleic acid molecule of any one of claims 1 to 3 or being transformed, transduced or transfected with the vector of claim 4.

6. The host cell of claim 5, wherein the host cell is a eukaryotic cell or a prokaryotic cell and is preferably a plant cell or an animal cell.

7. A plant, seed or a part of a plant, said part of a plant no being a single plant cell, or an animal comprising the nucleic acid molecule of any one of claims 1 to 3 or being transformed, transduced or transfected with the vector of claim 4.

8. A method of producing an RNA-guided DNA endonuclease comprising culturing the host cell of claim 5 or 6 and isolating the RNA-guided DNA endonuclease produced.

9. An RNA-guided DNA endonuclease encoded by the nucleic acid molecule of any one claims 1 to 3.

10. A composition comprising the nucleic acid molecule of any one of claims 1 to 3, the vector of claim 4, the host cell of claim 5 or 6, the plant, seed, part of a cell or animal of claim 7, the RNA-guided DNA endonuclease of claim 9 or a combination thereof.

11. The composition of claim 10, wherein the composition is a pharmaceutical composition or a diagnostic composition.

12. The nucleic acid molecule of any one of claims 1 to 3, the vector of claim 4, the host cell of claim 5 or 6, the plant, seed, part of a cell or animal of claim 7, the RNA-guided DNA endonuclease of claim 9 or a combination thereof for use in the treatment of a disease in a subject or a plant by modifying a nucleotide sequence at a target site in the genome of the subject or plant.

13. A method of modifying a nucleotide sequence at a target site in the genome of a cell comprising introducing into said cell
(i) a DNA-targeting RNA or a DNA polynucleotide encoding a DNA-targeting RNA, wherein the DNA-targeting RNA comprises:
(a) a first segment comprising a nucleotide sequence that is complementary to a sequence in the target DNA; and
(b) a second segment that interacts with the RNA-guided DNA endonuclease of claim 9; and
(ii) the RNA-guided DNA endonuclease of claim 9, or the nucleic acid molecule encoding an RNA-guided DNA endonuclease of any one of claims 1 to 3, or the vector of claim 4, wherein the RNA-guided DNA endonuclease comprises:
(a) an RNA-binding portion that interacts with the DNA-targeting RNA; and
(b) an activity portion that exhibits site-directed enzymatic activity.

14. The method of claim 13, wherein the cell is not the natural host of a gene encoding said RNA-guided DNA endonuclease.

15. The method of claim 13 or 14, wherein in case the RNA-guided DNA endonuclease and the DNA-targeting RNA are directly introduced into the cell they are introduced in the form of a ribonucleoprotein complex (RNP).


SEQUENCE LISTING

<110> B.R.A.I.N. Biotechnology Research And Information Network AG

<120> NOVEL CRISPR-Cas NUCLEASES FROM CPR-ENRICHED METAGENOME

<130> CCM_04

<160> 39

<170> BiSSAP 1.3

<210> 1
<211> 1161
<212> PRT
<213> Unknown

<220>
<223> Cps1 amino acid sequence

<400> 1
Met Gly Ile Ile His Tyr Lys Leu Gln Lys Gly Gly His Gly Asn Thr
1               5                   10                  15
Glu Ser Ala Phe Phe Lys Val Glu Ile Ala Ile Asp Trp Phe Ala Phe
            20                  25                  30
Asp Gly Arg Met Asp Arg Lys Gly Glu Ile Phe Ile Phe Ser Ser Ile
        35                  40                  45
Asn Phe Met Glu Thr Gln Ser Asn Phe Gly Lys Ile Gly Asn Leu Tyr
    50                  55                  60
Glu Thr Lys Lys Thr Ile Arg Phe Glu Leu Glu Pro Glu Phe Lys Asn
65                  70                  75                  80
Pro Pro Lys Ile Glu Lys Ser Lys Asn Phe Glu Glu Asp Leu Ile Ile
                85                  90                  95
Phe Leu Glu Lys Tyr Lys Arg Val Ile Leu Asn Phe Ser Lys Ile Val
            100                 105                 110
Phe Asn Asn Glu Gly Leu Asn Arg Gln Leu Lys Ile Asn Phe Ser Phe
        115                 120                 125
Leu Lys Thr His Thr Lys Arg Glu Tyr Phe Glu Asn Gly Val Glu Ala
    130                 135                 140
Leu Ile Lys Tyr Asn Lys Lys Gly Val Arg Ala Thr Asn Lys Ile Lys
145                 150                 155                 160
Ile Gly Asp Ser Ser Lys Leu Asn Tyr Leu Leu Glu Val Phe Lys Thr
                165                 170                 175
Ile Glu Ala Asn Asn Leu Glu Tyr Ile Lys Lys Ile Glu Gly Leu Gly
            180                 185                 190
Leu Arg Lys Glu Glu Asn Lys Ser Arg Lys Ser Asp Leu Ser Tyr Arg
        195                 200                 205
Phe Asn Gln Ile Ser Arg Lys Thr Asn Phe Asp Phe Ile Lys Asp Leu
    210                 215                 220
Phe Asp Asn Asn Ile Asn Thr Glu Gly Tyr Leu Ser Lys Asn Ile Glu
225                 230                 235                 240
Asn Ile Lys Glu Glu Leu Phe Glu Ile Lys Lys Ile Ile Leu Ser Ile
                245                 250                 255
Glu Lys Ser Leu Leu Pro Asn Gln Ser Met Gly Gln Val Ile Glu Lys
            260                 265                 270
Ser Ser Phe Asn Tyr Tyr Ile Val Asn Lys Lys Thr Arg Asn Tyr Asp
        275                 280                 285
Glu Glu Ile Leu Asp Lys Lys Ser Asn Phe Lys Lys Leu Leu Lys Glu
    290                 295                 300
Leu Asn Ile Ser Ile Glu Asp Ala Gly Phe Arg Lys Tyr Ile Ser Asn
305                 310                 315                 320
Thr Leu Ser Lys Thr Pro Thr Glu Leu Glu Ile Gln Val Ala Tyr Lys
                325                 330                 335

Leu Met Lys Glu Tyr Lys Ala Asp Gln Lys Gln Ala Phe Leu Gln Phe
            340                 345                 350
Leu Ser Lys Gly Tyr Asn Phe Lys Asp Leu Lys Asn Asn Ile Glu Phe
        355                 360                 365
Asn Leu Glu Leu Ile Asn Asn Glu Thr Gly Glu Lys Thr Thr Lys Lys
    370                 375                 380
Val Lys Ile Ser Leu Phe Asn Asp Ile Ser Glu Glu Asn Phe Lys Thr
385                 390                 395                 400
Met Phe Thr Leu Thr Asn Lys Ile Gln Lys Leu Ser Ala Glu Phe Asn
                405                 410                 415
Gln Thr Lys Asn Lys Thr Thr Lys Asp Glu Ile Thr Lys Leu Lys Lys
            420                 425                 430
Glu Lys Val Lys Tyr Phe Phe Glu Gly Asp Lys Leu Phe Ala Phe Lys
        435                 440                 445
Glu Tyr Gly Thr Phe Cys Lys Ser Tyr Lys Lys Val Ala Ile Ala Tyr
    450                 455                 460
Gly Arg Ile Lys Ala Asp Ile Lys Ser Leu Gly Lys Glu Lys Val Asp
465                 470                 475                 480
Ala Glu Arg Thr Asp Ser Trp Ala Leu Ile Leu Glu Asn Lys Asp Asn
                485                 490                 495
Lys Tyr Leu Leu Thr Ile Pro Arg Asn Gly Asn Lys Asp Lys Leu Ser
            500                 505                 510
Lys Ala Lys Ile Tyr Ile Asp Gly Leu Gln Asn Glu Ile Asn Gly Glu
        515                 520                 525
Asn Tyr Leu Tyr Lys Phe Glu Ser Leu Thr Leu Arg Ala Leu Asp Lys
    530                 535                 540
Leu Cys Phe Gly Lys Glu Asn Asn Thr Phe Arg Lys Ala Ile Tyr Leu
545                 550                 555                 560
Asp Arg Lys Thr Phe Pro Asn Phe Ser Asn Ser Asp Gly Lys Leu Lys
                565                 570                 575
Glu Lys Phe Glu Phe Lys Glu Asp Glu Lys Glu Leu Ile Asn Phe Tyr
            580                 585                 590
Lys Tyr Val Leu Asn Leu Glu Ser Thr Arg Lys Gln Ile Ala Leu Lys
        595                 600                 605
Tyr Phe Asn Gly Phe Asp Glu Phe Val Gln Arg Glu Phe Glu Thr Leu
    610                 615                 620
Tyr Lys Phe Glu Gly Glu Leu Lys Lys Ile Cys Tyr Thr Lys Glu Lys
625                 630                 635                 640
Ile Ile Ile Ser Asn Glu Thr Lys Asp Lys Ile Ile Asn Asp Tyr Asn
                645                 650                 655
Ala Lys Leu Tyr Lys Ile Thr Ser Tyr Asp Leu Lys Lys Asn Asp His
            660                 665                 670
Glu Tyr Ile Lys Asp Leu Glu Gln Lys Lys Gln Leu Asp Arg Gly Asn
        675                 680                 685
Pro Glu Pro His Thr Lys Ile Trp Arg Asp Phe Arg Thr Arg Asn Asn
    690                 695                 700
Glu Thr Asn Gln Tyr Gln Thr Arg Leu Asn Pro Glu Val Lys Ile Ser
705                 710                 715                 720
Phe Val Gly Lys Asp Glu Asp Phe Ile Lys Glu Asn Ile Lys Phe Ser
                725                 730                 735
Asn Asn Arg Arg Thr Lys Asp Arg Tyr Leu Leu Ser Thr Thr Ile Ser
            740                 745                 750
Gln Asn Ser Leu Gly Arg Tyr Ile Asp Leu Asn Phe Lys Glu Glu Ala
        755                 760                 765
Glu Ile Val Arg Phe Tyr Glu Glu Phe Asn Leu Asp Phe Asn Lys Asn
    770                 775                 780
Gln Lys Asn Asp Leu Asn Tyr Phe Tyr Gly Leu Asp Arg Gly Glu Asn
785                 790                 795                 800
Glu Leu Val Thr Leu Gly Ile Phe Asn Leu Thr Gln Lys Thr Asn Glu
                805                 810                 815
Asp Lys Gly Val Arg Ile Lys Val Phe Lys Leu Lys Asn Glu Ala Phe
            820                 825                 830
Leu Phe Ser Asp Gln Tyr Asn Lys Asp Glu Ser Pro Lys Leu Ala Tyr

835                 840                 845
Lys Asn Leu Ser Tyr Tyr Thr Tyr Asp Ser His Pro Asp Leu Tyr Glu
    850                 855                 860
Thr Glu Glu Val Ser Cys Ile Asp Leu Thr Thr Cys Lys Leu Ile Lys
865                 870                 875                 880
Gly Asn Ile Tyr Leu Asn Gly Asp Ile Gly Thr Tyr Leu Asn Leu Lys
                885                 890                 895
Leu Val Ser Ala Lys Arg Lys Ile Tyr Gly Ile Val Ser Leu Gly Lys
            900                 905                 910
Asn Lys Ser Asp Ile Ile Lys Ile Glu Asp Gly Tyr Lys Val Val Ile
        915                 920                 925
Lys Gly Tyr Asp Lys Glu Arg Gly Ser Ile Leu Tyr Ser Phe Asp Asp
    930                 935                 940
Lys Tyr Lys Asn Ile Gln Thr Lys Glu Lys Ile Ile Glu Ile Leu Gln
945                 950                 955                 960
Glu Tyr Leu Ile Lys Val Lys Asn Asn Leu Thr Asp Asn Glu Asn Ile
                965                 970                 975
Ser Ile Glu Lys Ile Asn Asn Leu Arg Asp Ser Ile Cys Ala Asn Met
            980                 985                 990
Val Gly Val Ile Asp Ser Leu Gln Gln Lys His Pro Gly Met Ile Phe
        995                 1000                1005
Phe Glu Asp Met Asp Ile Asn Ser Lys Asn Ser Phe Phe Asn Glu Asn
    1010                1015                1020
Asn Thr Thr Leu Gly Gly Arg Ile Glu Gln Lys Leu Leu Gln Lys Leu
1025                1030                1035                1040
Ser Ser Leu Asn Leu Val Pro Pro Asn Tyr Lys Leu Met Pro Thr Leu
                1045                1050                1055
Gln Lys Lys Ile Lys Gln Leu Gly Ile Ile Gly Tyr Val Asn Lys Glu
            1060                1065                1070
Asn Thr Ser Asn Ala Cys Pro Tyr Cys Lys Lys Asn Leu Phe Gly His
        1075                1080                1085
Gly Lys Gly Ser Asn Phe Glu Asn Ser Met His His Tyr Lys Glu Arg
    1090                1095                1100
Asn Gly Ile Asp Tyr Gly Asn Phe Leu Glu Gln Ala Lys Lys Thr Asn
1105                1110                1115                1120
Asn Asp Asn Cys Asp Tyr His Met Lys Asn Asn Asn Tyr Gly Phe Asp
                1125                1130                1135
Tyr Ile Lys Ser Gly Asp Asp Leu Ala Thr Tyr Asn Ile Ala Asn Arg
            1140                1145                1150
Gly Lys Glu Asp Ile Tyr Asp Lys Val
        1155                1160

<210> 2
<211> 3486
<212> DNA
<213> Unknown

<220>
<223> Cps1 native nucleotide sequence

<400> 2
atgggaataa tccattataa actacaaaaa ggctgacact gaaacacaga gtcagctttt      60

ttcaaagttg aaattgcaat tgattggttt gcttttgatg ggaggatgga tagaaaatga     120

gagatattta ttttctcctc aataaatttc atggaaacac aaagcaattt ctgaaaaata     180

tgaaatttat atgaaacaaa aaaaacaatc aggtttgaat tagaaccaga gtttaagaat     240

ccaccaaaaa tagaaaaatc taaaaatttt gaagaagact taattatctt tttagaaaaa     300

tataaaagag ttattttaaa cttctctaaa atagtattta acaatgaatg actaaataga     360

caactaaaaa taaattttag ctttctgaag acacacacaa aaagggaata ttttgaaaat 420

tgagttgaag cgttaataaa atacaacaaa aaatgagtta gagccacaaa taaaataaaa 480

atttgagaca gttctaaatt aaattattta ctcgaagtat ttaaaacaat tgaagcaaac 540

aatttagaat atataaaaaa aatagaagga ttgtgattaa gaaaagaaga aaataaatct 600

agaaaatctg atttatcata tcgatttaat caaatatcta gaaaaacaaa ttttgatttt 660

ataaaagatc tttttgataa taatataaat actgaatgat atctatctaa aaatattgaa 720

aacataaaag aagagttatt tgaaattaaa aaaataattt taagtataga aaaaagccta 780

ttaccaaatc aatcaatggg acaagtcata gagaagtcaa gctttaacta ttatattgta 840

aataaaaaaa caagaaatta tgatgaggaa atcttagaca agaaaagcaa ctttaaaaaa 900

ttattaaaag agctaaatat aagtatagaa gatgcaggat ttaggaaata tatctctaat 960

actttaagta aaacgccaac tgaattagaa atccaagtag cttataaact tatgaaagaa 1020

tacaaggcag atcaaaaaca agcattttta caatttctat caaaaggata taacttcaaa 1080

gatttaaaaa acaatataga atttaacctt gaattaataa ataatgaaac atgagagaaa 1140

acgaccaaaa aggtaaaaat atcattattt aacgatatct cagaagaaaa ttttaaaacg 1200

atgttcactc taaccaataa aatacaaaaa ctatctgctg agttcaatca aaccaaaaat 1260

aagacaacaa aagatgaaat tactaaacta aaaaaagaaa aggtaaaata tttttttgaa 1320

tgagataaat tattcgcatt taaagaatat ggtacttttt gcaaatctta caaaaaggta 1380

gcaatagcat attgaagaat caaagcagat attaaatcac taggaaaaga aaaagttgat 1440

gcagaaagaa ctgacagttg ggctttgatt ttagaaaaca aagacaataa atatctatta 1500

accataccaa gaaatggaaa taaagataag ttaagtaagg caaaaatata tattgatggt 1560

ttacagaacg agattaacgg agaaaattat ttatataaat ttgaatcttt aacactaaga 1620

gcactagata agctttgttt ctgaaaagaa aacaacacct ttagaaaggc tatctattta 1680

gatagaaaga catttcctaa tttttccaat agcgatggga agttaaaaga gaagtttgaa 1740

tttaaggaag atgaaaaaga attgattaac ttttacaaat atgtgttaaa cttagaatca 1800

acaagaaaac aaatagcatt aaaatacttc aatggatttg atgaatttgt tcaaagagaa 1860

tttgagactc tatataaatt tgaaggagag ttaaaaaaaa tttgctatac gaaagaaaaa 1920

ataatcattt caaatgaaac aaaagacaag ataataaatg attacaatgc taaattatat 1980

aaaattacca gttatgattt gaagaaaaat gaccatgaat atattaaaga tctagaacaa 2040

aaaaaacaat tagacaggtg aaatcccgaa ccacatacaa aaatttggcg agattttcga 2100

acaaggaaca acgaaacaaa tcaataccaa acaagattaa atccagaagt taaaattagc 2160

tttgtgggaa aagatgaaga ttttatcaaa gaaaatataa aatttagtaa taatagaagg 2220

acaaaagata gatatttact aagcacaacg ataagtcaaa attcactagg gagatacatt 2280

gatctaaatt ttaaagaaga agcagaaatt gtaagatttt atgaagaatt taatctcgat 2340

tttaataaaa accaaaaaaa tgatttaaac tatttttatt gattagatag atgagaaaat 2400

gaacttgtaa ccttatgaat atttaattta actcagaaaa cgaatgaaga taaatgagtg 2460

agaattaaag tatttaaatt aaaaaatgaa gcattccttt ttagcgatca atacaataaa 2520

gacgaatctc caaagttagc gtataagaac ctatcgtact atacatacga tagccaccct 2580

gatttgtatg aaacagaaga ggtgtcttgt attgatctca ccacctgtaa attaatcaaa 2640

tgaaatattt atctaaactg agatatttga acatatctaa atctaaaatt agtttcagcg 2700

aaaagaaaaa tttacggaat cgtatctctt ggaaaaaaca aatcagatat tattaaaata 2760

gaggatggtt ataaagtagt tattaaatga tacgataaag aaagatgatc aattttatat 2820

tcttttgatg acaaatataa gaatattcag actaaagaaa aaattatcga gattctacaa 2880

gaatatttga taaaagtcaa aaacaatcta actgataatg aaaatatttc cattgaaaag 2940

ataaataatc taagagattc aatttgtgca aacatggtct gagttataga ttctttgcaa 3000

caaaaacatc cttgaatgat atttttcgaa gatatggata taaacagcaa gaacagtttt 3060

ttcaacgaaa ataacacaac acttggatga agaatcgaac aaaaattatt acagaagctt 3120

tcttccctaa atctagttcc tccaaactat aaactaatgc ccacattaca aaaaaaaatt 3180

aaacaattat gaataatttg atatgtaaat aaagaaaaca cttctaatgc ttgtccatat 3240

tgcaagaaaa accttttttg acactgaaaa tgatcgaatt ttgaaaatag catgcatcat 3300

tacaaagaga gaaacggaat tgattattga aattttttag aacaggctaa aaaaacaaac 3360

aatgataatt gcgattatca tatgaaaaat aataattatt gatttgatta tattaagtca 3420

ggagatgatc tagctacata taatattgca aatagaggga aagaagatat atatgacaaa 3480

gtataa 3486

<210> 3
<211> 1034
<212> PRT
<213> Unknown

<220>
<223> Cps2 amino acid sequence

<400> 3
Lys Asn Leu Ser Asn Arg Asn Lys Glu Asn Lys Ala Arg Lys Ser Asp
1               5                   10                  15
Leu Gly Val Tyr Phe Asn Arg Leu Thr Lys Arg Thr Asn Phe Glu Phe
            20                  25                  30
Val Arg Glu Leu Phe Asn Asn Asn Ile Gly Ile Glu Asn Lys Asp Ile
        35                  40                  45
Ala Lys Lys Ile Asp Glu Thr Lys Ile Ile Thr Gln Glu Ser Gln Asn
    50                  55                  60
Leu Ile Leu Lys Ile Gln Lys Thr Leu Leu Pro Asn Glu Ser Ile Gly
65                  70                  75                  80

Gln Val Ile Glu Lys Ala Ser Phe Asn Tyr Tyr Thr Val Asn Lys Arg
                85                  90                  95
Lys Arg Asp Tyr Asp Glu Met Ile Lys Asn Lys Glu Asn Asp Lys Asn
            100                 105                 110
Lys Lys Leu Lys Asp Phe Tyr Pro Val Leu Ser Ile Glu Asp Lys Gly
        115                 120                 125
Phe Lys Ile Phe Thr Ser Ser Ile Thr Asp Lys Thr Gly Asn Leu Glu
    130                 135                 140
Ile Glu His Ala Tyr Asn Leu Met Lys Glu Tyr Lys Ala Glu Gln Lys
145                 150                 155                 160
Ser Asn Phe Leu Glu Phe Leu Ala Glu Lys Ser Lys Gly Asn Glu Ser
                165                 170                 175
Asn Leu Asp Ile Ser Leu Phe Tyr Glu Ile Leu Asp Lys Ser Gln Tyr
            180                 185                 190
Lys Glu Ile Ile Asn Leu Thr Gln Ala Ile Leu Leu Leu Ser Gln Val
        195                 200                 205
Lys Thr Asp Ile Ile Asn Phe Lys Asp Thr Asn Asn Lys Glu Lys Lys
    210                 215                 220
Glu Glu Glu Ile Gln Gly Asn Leu Glu Lys Tyr Asn Ile Glu Phe Gly
225                 230                 235                 240
Asn Asp Phe Gly Lys Val Lys Lys Asp Asn Phe Phe Asp Lys Ile Thr
                245                 250                 255
Glu Leu Lys Lys Leu Arg Gly Glu Tyr Phe Phe Glu Gly Asp Arg Thr
            260                 265                 270
Gly Lys Phe Ala Phe Lys Lys Tyr Gly Lys Phe Cys Glu Asp Tyr Lys
        275                 280                 285
Lys Val Ala Ile Glu Leu Gly Arg Ile Lys Ala Asn Ile Lys Ser Leu
    290                 295                 300
Glu Lys Glu Lys Asn Asp Ala Glu Lys Thr Gln Ser Trp Ala Leu Ile
305                 310                 315                 320
Leu Glu Lys Asp Asn Asn Lys Tyr Leu Leu Thr Ile Pro Arg Asn Lys
                325                 330                 335
Lys Asp Ser Ser Gly Glu Asn Phe Glu Thr Asn Leu Gln Lys Ser Lys
            340                 345                 350
Lys Glu Ile Asp Ser Leu Glu Lys Ile Asn Ser Gly Glu Leu Val Leu
        355                 360                 365
Tyr Lys Phe Glu Ser Leu Thr Leu Arg Ala Leu Asp Lys Leu Cys Phe
    370                 375                 380
Gly Lys Glu Asn Asn Thr Phe Ala Gly Asn Ile Ala Lys Glu Leu Phe
385                 390                 395                 400
Glu Lys Asn Asn Asn Phe Val Glu Lys Lys Thr Arg Gly Glu Lys Asn
                405                 410                 415
Phe Tyr Asn Leu Lys Arg Lys Asp Glu Ile Gly Asn Lys Asp Asp Lys
            420                 425                 430
Phe Lys Asn Asp Lys Asn Leu Ile Ser Phe Tyr Lys Thr Ile Leu Ser
        435                 440                 445
Leu Gln Ser Thr Gln Lys Gln Ile Ile Val Thr His Phe Lys Asp Phe
    450                 455                 460
Asp Thr Phe Ile Lys Thr Glu Phe Gln Asn Leu Glu Asp Phe Glu Arg
465                 470                 475                 480
Glu Leu Lys Lys Thr Cys Tyr Ile Lys Asp Ser Phe Cys Ile Ser Glu
                485                 490                 495
Asp Gln Lys Asn Glu Ile Ile Lys Asn Phe Glu Ala Lys Leu Tyr Lys
            500                 505                 510
Ile Thr Ser Tyr Asp Leu Gln Lys Asn Asp Thr Glu Phe Phe Lys Ala
        515                 520                 525
Leu Asn Glu Glu Lys Gln Lys Thr Gly Leu Lys Arg Leu Lys Thr Lys
    530                 535                 540
Asp Asn Asp Val Thr Asn Leu Trp Leu Ser Phe Trp Gly Ser Asp Asn
545                 550                 555                 560
Glu Lys Asn Lys Tyr Pro Ile Arg Leu Asn Pro Glu Ile Ser Ile Thr
                565                 570                 575
Tyr Leu Glu Ala Asp Asp Lys Phe Thr Gln Asn Asn Ser Gly Leu Lys

580                 585                 590
Tyr Asn Arg Lys Phe His Asp Arg Tyr Leu Leu Ser Thr Thr Ile Thr
        595                 600                 605
Glu Lys Ala Leu Glu Lys Ser Leu Asp Leu Ser Phe Lys Glu Lys Ala
    610                 615                 620
Asp Ile Ile Lys Val Tyr Glu Asp Tyr Asn Ile Asn Phe Asn Gln Glu
625                 630                 635                 640
Asn Lys Phe Gln Tyr Ile Tyr Gly Leu Asp Arg Gly Glu Asn Glu Leu
                645                 650                 655
Val Ser Leu Gly Val Phe Asp Leu His Lys Glu Lys Ile Glu Glu Arg
            660                 665                 670
Gly Val Lys Ile Lys Val Tyr Glu Leu Lys Lys Asp Cys Phe Leu Lys
        675                 680                 685
Pro Asp Glu Lys Gly Arg Ile Ala Tyr Lys Asn Leu Ser Tyr Phe Gln
    690                 695                 700
Phe Asp Thr Tyr Pro Glu Phe Tyr Glu Lys Glu Lys Glu Val Ser Cys
705                 710                 715                 720
Ile Asp Leu Thr Thr Ser Lys Leu Ile Asn Gly Lys Ile Tyr Leu Asn
                725                 730                 735
Gly Asp Ile Gln Thr Tyr Leu Asn Leu Lys Leu Leu Ala Ala Lys Arg
            740                 745                 750
Lys Ile Tyr Glu Ala Val Ser Gln Lys Phe Leu Gly Asp Glu Glu Ile
        755                 760                 765
Lys Thr Cys Lys Tyr Asn Val Leu Lys Leu Ser Ser Gln Ile Glu Glu
    770                 775                 780
Glu Lys Leu Glu Gln Gln Glu Leu Asp Lys Lys Gly Lys Asn Glu Lys
785                 790                 795                 800
Val Lys Asp Lys Tyr Ile Tyr Lys Phe Asp Glu Arg Tyr Asp Asn Ile
                805                 810                 815
Phe Ser Phe Glu Asn Thr Lys Glu Glu Leu Asn Lys Tyr Leu Lys Lys
            820                 825                 830
Val Gln Glu Asn Leu Ser Asp Asn Glu Glu Val Ser Ile Gln Lys Val
        835                 840                 845
Asn Asn Leu Arg Asp Ala Ile Cys Ala Asn Met Ile Gly Ile Ile Lys
    850                 855                 860
Phe Leu Gln Asp Glu Tyr Ser Gly Met Ile Thr Leu Glu Asp Leu Thr
865                 870                 875                 880
Ile Asp Trp Lys Asn Asn Thr Phe Asp Lys Ser Asn Thr Thr Leu Ser
                885                 890                 895
Ser Arg Phe Glu Gln Lys Leu Leu Gln Lys Phe Ser Ser Leu Asn Leu
            900                 905                 910
Val Pro Pro Thr Tyr Lys Gln Ile Leu Ser Ile Lys Asp Glu Lys Asn
        915                 920                 925
Thr Ser Ile Lys Gln Leu Gly Ile Ile Ser Tyr Val Asn Lys Asp Gly
    930                 935                 940
Thr Ser Thr Thr Cys Pro Asp Cys Glu Lys Lys Leu Phe Gly His Gly
945                 950                 955                 960
Lys Gly Pro Asp Phe Glu Asn Ser Met His His Tyr Glu Glu Asn Asn
                965                 970                 975
Gly Ile Asp Tyr Gly Asp Asp Leu Glu Glu Ala Arg Lys Thr Gln Asn
            980                 985                 990
Pro Asp Cys Asp Tyr His Met Pro Asp Glu Lys Tyr Gly Phe Thr Phe
        995                 1000                1005
Ile Lys Ser Gly Asp Asp Leu Ala Thr Tyr Asn Ile Ala Lys Lys Gly
    1010                1015                1020
Leu Glu Phe Ile Lys Thr Gln Asn Lys Ser
1025                1030

<210> 4
<211> 3105
<212> DNA
<213> Unknown

<220>
<223> Cps2 native nucleotide sequence

<400> 4
aaaaatcttt caaatagaaa taaagaaaat aaagctagaa aatcagattt aggagtatat 60

tttaatagac ttacaaaaag aacaaatttt gagtttgtca gagaactttt taataataat 120

atttgaattg aaaataaaga tattgctaaa aaaatagatg aaacaaaaat tattactcaa 180

gaatcacaaa accttatttt aaaaattcaa aaaactttgc ttccaaatga gtcaatttga 240

caagttatag aaaaagcaag ttttaattac tatactgtaa ataaaagaaa aagagattat 300

gatgaaatga taaaaaataa agaaaatgat aagaacaaaa aattaaaaga cttttatcct 360

gttttaagta tagaagataa aggatttaaa atttttacct caagcatcac agataaaact 420

tgaaatcttg aaatagaaca tgcttataat cttatgaaag aatataaggc agaacaaaaa 480

tcaaatttct tagagttttt agctgaaaaa tcaaaatgaa atgagtcaaa tttagatatc 540

tctttatttt atgaaatttt agataaatct caatacaaag aaattataaa tttaacacaa 600

gcaattttat tattatctca agtaaaaaca gatattataa actttaaaga tactaataat 660

aaagagaaaa aggaagaaga aattcaaggt aatttagaaa aatataatat tgagttttga 720

aatgattttt gaaaagtaaa gaaagataac ttttttgata aaataactga actaaaaaaa 780

ctaagatgag agtatttttt tgaatgagat agaacttgaa aatttgcttt taaaaaatat 840

ggtaaatttt gtgaagatta taaaaaagtt gcaatagaac tttgaagaat aaaagcaaat 900

ataaaatctc ttgaaaaaga aaaaaatgat gcagaaaaaa ctcaaagttg ggctttaatt 960

ttagaaaaag ataataataa atatttactt acaattccaa gaaataaaaa agatagttct 1020

tgagaaaatt ttgaaacaaa tctgcaaaaa tctaaaaagg aaatagatag tttagaaaaa 1080

ataaattctg gtgaattagt tttatataaa tttgaatctt tgactttaag agctttagat 1140

aaactttgtt tttgaaagga aaataacact tttgcttgaa atatagcaaa agaacttttt 1200

gaaaaaaata ataatttcgt tgagaaaaaa actagatgag aaaaaaattt ttacaatcta 1260

aaaagaaaag atgaaatttg aaataaagat gataaattta aaaatgataa aaatctaata 1320

agtttctata aaacaatttt atctttacaa tcaactcaaa aacaaattat tgttactcat 1380

tttaaagatt ttgatacatt tataaaaaca gagtttcaaa atttagaaga ttttgaaaga 1440

gaactcaaaa aaacttgtta cataaaagat agtttttgca tttcagaaga tcaaaaaaat 1500

gaaatcataa aaaattttga agcaaaactt tataaaatca caagttatga tttacaaaaa 1560

aatgatacag aattttttaa agctttaaat gaagaaaaac aaaaaacttg attaaaaaga 1620

ctcaaaacaa aagataatga tgtaacaaat ctttggctta gtttttggtg aagtgataat 1680

gaaaaaaata aatatcctat aagactcaat ccagaaataa gtataactta tttagaagca 1740

gatgataagt ttactcaaaa taattcatga ctgaaatata atagaaaatt tcatgataga 1800

tatttgctct caactactat tacagaaaaa gctttggaaa aatctttaga tttaagtttt 1860

aaagaaaaag ctgatattat taaagtttat gaagattata atataaactt taatcaagaa 1920

aataaatttc aatatattta ttgacttgat agatgagaaa atgaacttgt aagtttatga 1980

gtttttgatt tacacaaaga aaaaatagaa gaaagatgag taaaaataaa agtttatgaa 2040

ttaaaaaaag attgcttttt aaagccagat gaaaaatgaa gaattgcata taaaaatctt 2100

tcatattttc aatttgatac atatcctgag ttttatgaaa aagaaaaaga agtttcttgt 2160

atagatctta caacttctaa acttataaat tgaaaaattt atcttaattg agatattcaa 2220

acttatttga atctaaaact tcttgcagca aaaagaaaga tttatgaagc agtttctcaa 2280

aaatttttgt gagatgaaga aatcaaaact tgtaaatata atgtactcaa attatcttct 2340

caaattgaag aggaaaaact tgaacaacaa gaacttgata aaaaatgaaa gaatgaaaaa 2400

gtaaaagaca aatatatcta taaatttgat gaaagatatg ataatatttt ttcttttgaa 2460

aatactaaag aagaactaaa taaatatctt aaaaaagtac aagaaaattt aagtgataat 2520

gaagaagttt caattcaaaa agttaataat cttagagatg caatttgcgc aaatatgatt 2580

tgaataataa aatttttaca agatgaatat tcttgaatga ttactttaga agatttaaca 2640

atagattgga aaaataatac ttttgataaa agcaatacta cattatcaag tagatttgaa 2700

caaaaattat tacaaaaatt ttcttcacta aatttagttc caccaactta taaacaaatt 2760

ctatctataa aagatgaaaa aaatactagt ataaaacaac tttgaattat ctcttatgta 2820

aataaggatt gaacatctac aacgtgtcca gattgtgaaa aaaaactttt ttgacactga 2880

aaatgaccag attttgaaaa tagtatgcat cattatgaag aaaataattg aatagattat 2940

tgagatgatt tagaagaagc tagaaaaact caaaatcctg attgtgatta tcatatgcca 3000

gatgaaaagt attgatttac ttttataaaa tcttgagacg atctcgcaac ctataacatc 3060

gcaaaaaaag gtttagagtt tataaaaaca caaaataaat cataa 3105

<210> 5
<211> 2026
<212> DNA
<213> Triticum aestivum

<220>
<223> GUS gene

<400> 5
atggatccct acagggtaaa tttctagttt ttctccttca ttttcttggt taggaccctt 60

ttctcttttt atttttttga gctttgatct ttctttaaac tgatctattt tttaattgat 120

tggttatggt gtaaatatta catagcttta actgataatc tgattacttt atttcgtgtg 180

tctatgatga tgatgatagt tacagaaccg tcgactcgtc cgtcctgtag aaaccccaac 240

ccgtgaaatc aaaaaactcg acggcctgtg ggcattcagt ctggatcgcg aaaactgtgg 300

aattgatcag cgttggtggg aaagcgcgtt acaagaaagc cgggcaattg ctgtgccagg 360

cagttttaac gatcagttcg ccgatgcaga tattcgtaat tatgcgggca acgtctggta 420

tcagcgcgaa gtctttatac cgaaaggttg ggcaggccag cgtatcgtgc tgcgtttcga 480

tgcggtcact cattacggca aagtgtgggt caataatcag gaagtgatgg agcatcaggg 540

cggctatacg ccatttgaag ccgatgtcac gccgtatgtt attgccggga aaagtgtacg 600

tatcaccgtt tgtgtgaaca acgaactgaa ctggcagact atcccgccgg gaatggtgat 660

taccgacgaa aacggcaaga aaaagcagtc ttacttccat gatttcttta actatgccgg 720

aatccatcgc agcgtaatgc tctacaccac gccgaacacc tgggtggacg atatcaccgt 780

ggtgacgcat gtcgcgcaag actgtaacca cgcgtctgtt gactggcagg tggtggccaa 840

tggtgatgtc agcgttgaac tgcgtgatgc ggatcaacag gtggttgcaa ctggacaagg 900

cactagcggg actttgcaag tggtgaatcc gcacctctgg caaccgggtg aaggttatct 960

ctatgaactg tgcgtcacag ccaaaagcca gacagagtgt gatatctacc cgcttcgcgt 1020

cggcatccgg tcagtggcag tgaagggcga acagttcctg attaaccaca aaccgttcta 1080

ctttactggc tttggtcgtc atgaagatgc ggacttgcgt ggcaaaggat tcgataacgt 1140

gctgatggtg cacgaccacg cattaatgga ctggattggg gccaactcct accgtacctc 1200

gcattaccct tacgctgaag agatgctcga ctgggcagat gaacatggca tcgtggtgat 1260

tgatgaaact gctgctgtcg gctttaacct ctctttaggc attggtttcg aagcgggcaa 1320

caagccgaaa gaactgtaca gcgaagaggc agtcaacggg gaaactcagc aagcgcactt 1380

acaggcgatt aaagagctga tagcgcgtga caaaaaccac ccaagcgtgg tgatgtggag 1440

tattgccaac gaaccggata cccgtccgca aggtgcacgg gaatatttcg cgccactggc 1500

ggaagcaacg cgtaaactcg acccgacgcg tccgatcacc tgcgtcaatg taatgttctg 1560

cgacgctcac accgatacca tcagcgatct ctttgatgtg ctgtgcctga accgttatta 1620

cggatggtat gtccaaagcg gcgatttgga aacggcagag aaggtactgg aaaaagaact 1680

tctggcctgg caggagaaac tgcatcagcc gattatcatc accgaatacg gcgtggatac 1740

gttagccggg ctgcactcaa tgtacaccga catgtggagt gaagagtatc agtgtgcatg 1800

gctggatatg tatcaccgcg tctttgatcg cgtcagcgcc gtcgtcggtg aacaggtatg 1860

gaatttcgcc gattttgcga cctcgcaagg catattgcgc gttggcggta acaagaaagg 1920

gatcttcact cgcgaccgca aaccgaagtc ggcggctttt ctgctgcaaa aacgctggac 1980

tggcatgaac ttcggtgaaa aaccgcagca gggaggcaaa caatga 2026

<210> 6
<211> 2081

<212> DNA
<213> Artificial Sequence

<220>
<223> Mutant GUS gene

<400> 6
atggatccct acagggtaaa tttctagttt ttctccttca ttttcttggt taggaccctt 60

ttctcttttt atttttttga gctttgatct ttctttaaac tgatctattt tttaattgat 120

tggttatggt gtaaatatta catagcttta actgataatc tgattacttt atttcgtgtg 180

tctatgatga tgatgatagt tacagaaccg tcgactcgtc cgtcctgtag aaaccccaac 240

ccgtgaaatc aaaaaactcg acggcctgtg ggcattcagt ctggatcgcg aaaactgtgg 300

aattgatcag cgttggtggg aaagcgcgtt acaagaaagc cgggcaattg ctgtgccagg 360

cagttttaac gatcagttcg ccgatgcaga tattcgtaat tatgcgggca acgtctggta 420

tcagcgcgaa gtctttatac cgaaaggttg ggcaggccag cgtatcgtgc tgcgtttcga 480

tgcggtcacg taagtagctg aggcgggcag gccagcgtat cgtgctgcgt ttcgatgcgg 540

tcactcatta cggcaaagtg tgggtcaata atcaggaagt gatggagcat cagggcggct 600

atacgccatt tgaagccgat gtcacgccgt atgttattgc cgggaaaagt gtacgtatca 660

ccgtttgtgt gaacaacgaa ctgaactggc agactatccc gccgggaatg gtgattaccg 720

acgaaaacgg caagaaaaag cagtcttact tccatgattt ctttaactat gccggaatcc 780

atcgcagcgt aatgctctac accacgccga acacctgggt ggacgatatc accgtggtga 840

cgcatgtcgc gcaagactgt aaccacgcgt ctgttgactg gcaggtggtg gccaatggtg 900

atgtcagcgt tgaactgcgt gatgcggatc aacaggtggt tgcaactgga caaggcacta 960

gcgggacttt gcaagtggtg aatccgcacc tctggcaacc gggtgaaggt tatctctatg 1020

aactgtgcgt cacagccaaa agccagacag agtgtgatat ctacccgctt cgcgtcggca 1080

tccggtcagt ggcagtgaag ggcgaacagt tcctgattaa ccacaaaccg ttctacttta 1140

ctggctttgg tcgtcatgaa gatgcggact tgcgtggcaa aggattcgat aacgtgctga 1200

tggtgcacga ccacgcatta atggactgga ttggggccaa ctcctaccgt acctcgcatt 1260

acccttacgc tgaagagatg ctcgactggg cagatgaaca tggcatcgtg gtgattgatg 1320

aaactgctgc tgtcggcttt aacctctctt taggcattgg tttcgaagcg ggcaacaagc 1380

cgaaagaact gtacagcgaa gaggcagtca acggggaaac tcagcaagcg cacttacagg 1440

cgattaaaga gctgatagcg cgtgacaaaa accacccaag cgtggtgatg tggagtattg 1500

ccaacgaacc ggatacccgt ccgcaaggtg cacgggaata tttcgcgcca ctggcggaag 1560

caacgcgtaa actcgacccg acgcgtccga tcacctgcgt caatgtaatg ttctgcgacg 1620

ctcacaccga taccatcagc gatctctttg atgtgctgtg cctgaaccgt tattacggat 1680

ggtatgtcca aagcggcgat ttggaaacgg cagagaaggt actggaaaaa gaacttctgg 1740

cctggcagga gaaactgcat cagccgatta tcatcaccga atacggcgtg gatacgttag 1800

ccgggctgca ctcaatgtac accgacatgt ggagtgaaga gtatcagtgt gcatggctgg 1860

atatgtatca ccgcgtcttt gatcgcgtca gcgccgtcgt cggtgaacag gtatggaatt 1920

tcgccgattt tgcgacctcg caaggcatat tgcgcgttgg cggtaacaag aaagggatct 1980

tcactcgcga ccgcaaaccg aagtcggcgg cttttctgct gcaaaaacgc tggactggca 2040

tgaacttcgg tgaaaaaccg cagcagggag gcaaacaatg a 2081

<210> 7
<211> 3222
<212> DNA
<213> Artificial Sequence

<220>
<223> SmCms1 gene (plant codon optimized)

<400> 7
atggccccaa agaagaagcg caaggtgatg gaaaagtaca agatcaccaa gaccatcagg 60

ttcaagctgc tcccagacaa gatccaagac atctccaggc aagtggccgt gctccaaaac 120

tccaccaacg ccgagaagaa gaacaacctc ctcaggctcg tgcaaagggg ccaagagctt 180

ccaaagctcc tcaacgagta catcaggtac tccgacaacc acaagctcaa gtccaacgtg 240

accgtgcact tccgctggct caggctgttc accaaggacc tcttctacaa ctggaagaag 300

gacaacaccg aaaagaagat caagatctcc gacgtggtgt acctcagcca cgtgttcgag 360

gcctttctca aggaatggga gtccaccatc gagagggtga acgccgactg caacaagcca 420

gaggaatcca agaccaggga cgccgagatc gccctctcca ttaggaagct cggcatcaag 480

caccaactgc cattcatcaa gggcttcgtg gacaactcca acgacaagaa ctccgaggac 540

accaagtcca agctcaccgc tctgctctcc gagttcgagg cggttctgaa gatctgcgag 600

caaaactacc tgccaagcca atcctccgga atcgcgatcg ccaaggcctc cttcaactac 660

tacaccatca acaagaagca aaaggacttc gaggctgaga tcgtggccct gaagaagcaa 720

ctccacgcca ggtacgggaa caagaagtac gaccaactcc tccgcgagct gaacctcatt 780

ccactcaagg aactcccgct gaaggaactg ccactgatcg agttctactc cgagatcaag 840

aagaggaagt ccaccaagaa gtccgagttc ctcgaggccg tgtctaacgg cctcgtgttc 900

gacgacctca agagcaagtt cccactcttc caaaccgagt ccaacaagta cgacgagtac 960

ctcaagctca gcaacaagat tacccaaaag tctaccgcca agagcctgct ctccaaggac 1020

tccccagagg cgcaaaagct gcaaaccgag atcactaagc tcaagaagaa ccgcggcgag 1080

tacttcaaga aggcgttcgg caagtacgtg caactctgcg agctgtacaa ggaaatcgcc 1140

ggcaagaggg gcaagctgaa gggccaaatc aagggcatcg agaacgagag gatcgactcc 1200

caaaggctcc aatactgggc cctcgtgctc gaggacaacc tcaagcactc cctcattctc 1260

atcccaaagg aaaagaccaa cgagctttac cgcaaggtgt ggggcgccaa ggatgatggc 1320

gcatcctctt catccagcag caccctctac tacttcgagt ccatgaccta cagggccctc 1380

cgcaagctct gcttcggaat caacggcaac accttcctgc cagagatcca aaaggaactt 1440

ccacagtaca accagaagga attcggcgag ttctgcttcc acaagagcaa cgacgataag 1500

gaaattgacg agccaaagct catctccttc taccaatccg tgctcaagac cgacttcgtg 1560

aagaacaccc tcgcgctccc acaaagcgtg ttcaacgagg tggccatcca atccttcgag 1620

actaggcaag acttccagat cgcgctcgag aagtgctgct acgccaagaa gcagatcatc 1680

tccgagagcc tcaagaagga aatcctcgag aactacaaca cccaaatctt caagatcact 1740

tccctcgacc tccaaaggtc cgagcaaaag aacctcaagg gccacaccag gatctggaac 1800

aggttctgga ccaagcaaaa cgaggaaatc aactacaacc tcaggctgaa cccagagatc 1860

gccatcgttt ggaggaaggc caagaagacc aggatcgaga agtacggcga gcgctccgtt 1920

ctctacgagc cagaaaagag gaaccgctac ctccacgagc agtacaccct ctgcaccacc 1980

gttaccgaca acgccctcaa caacgagatc accttcgcct tcgaggatac caagaagaag 2040

ggcaccgaga tcgtcaagta caacgaaaag atcaatcaaa ccctgaagaa ggaattcaac 2100

aagaaccagc tgtggttcta cggcatcgac gccggcgaga tcgagcttgc tactctcgcc 2160

ctcatgaaca aggacaagga accacaactc ttcaccgtgt acgagcttaa gaagctggac 2220

ttcttcaagc acggctacat ctacaacaag gaacgcgagc tggtgatccg cgagaagcca 2280

tacaaggcga tccaaaacct ctcctacttc ctgaacgagg aactgtacga aaagaccttc 2340

cgcgacggca agtttaacga gacttacaac gagttgttca aggaaaagca cgtcagcgcc 2400

atcgacctca ccaccgccaa ggtgatcaac gggaagatca tcctcaacgg cgacatgatc 2460

acattcctca acctccgcat cctgcacgcc caacgcaaga tctatgagga acttatcgag 2520

aacccacacg ccgaactgaa ggaaaaggat tacaagctgt acttcgagat cgaaggcaag 2580

gataaggaca tctacatctc ccgcctcgac ttcgagtaca ttaagccata ccaagagatc 2640

agcaactacc tcttcgccta cttcgccagc caacaaatca acgaggcccg cgaggaagaa 2700

cagatcaacc agaccaagag ggcgctcgcg ggcaacatga tcggcgtgat ctactacctc 2760

taccaaaagt acaggggcat catctccatc gaggacctga agcagaccaa ggtcgagagc 2820

gaccgcaaca agttcgaggg caacatcgag cgcccattgg agtgggccct ctaccgcaag 2880

ttccaacaag agggctacgt gccaccaatc tccgagctga tcaagctccg cgaacttgag 2940

aagttcccgc tcaaggacgt caagcagcca aagtacgaga acatccaaca attcggcatc 3000

atcaagttcg tgagcccaga ggaaacctcc accacctgtc caaagtgcct caggcgcttc 3060

aaggactacg ataagaacaa gcaagaggga ttctgcaagt gccaatgcgg cttcgacacc 3120

cgcaacgacc tgaagggatt cgagggcctc aacgacccag ataaggtggc cgccttcaac 3180

attgccaagc ggggctttga ggacctgcaa aagtataagt ga 3222

<210> 8
<211> 3567
<212> DNA
<213> Artificial Sequence

<220>
<223> Cps1 gene (plant codon optimized)

<400> 8
atggccccga agaaaaagcg caaggtgatg ggcatcatcc actacaagct ccagaaaggc 60

ggccacggca ataccgagtc cgccttcttc aaggtcgaga tcgccatcga ctggttcgcc 120

ttcgacggcc gcatggatag gaagggcgag atcttcatct tctcctccat caacttcatg 180

gaaacccagt ccaacttcgg caagatcggc aacctctacg agacaaagaa aaccatccgc 240

ttcgagcttg agccagagtt caagaacccg ccgaagatcg agaagtccaa gaacttcgaa 300

gaggacctga tcatcttcct cgagaagtat aagcgcgtga tcctcaactt ctccaagatc 360

gtgttcaaca acgagggcct caaccgccag ctcaagatca atttcagctt cctcaagacc 420

cacaccaagc gcgagtactt cgagaatggc gtcgaggccc tcatcaagta caacaagaaa 480

ggcgtgcgcg ccaccaacaa gatcaagatt ggcgactcct ccaagctcaa ctacctcctc 540

gaggtgttca agaccatcga ggccaacaac ctcgagtaca tcaagaagat cgaaggcctc 600

ggcctccgca aagaggaaaa caagtcccgc aagtccgacc tctcctaccg cttcaatcag 660

atctcccgca agaccaactt cgacttcatc aaggacctct tcgacaacaa catcaacaca 720

gagggctacc tcagcaagaa catcgagaat atcaaagaag aactcttcga gattaagaag 780

atcatcctct ccattgagaa gtccctgctg ccgaaccaga gcatgggcca agtgatcgaa 840

aagtcctcct tcaactacta catcgtgaac aagaaaaccc gcaactacga cgaggaaatc 900

ctcgacaaga agtcgaactt caagaagctc ctcaaagagc tgaacatctc catcgaggac 960

gccggcttcc gcaagtacat cagcaacacc ctctccaaga cgccgaccga actcgagatt 1020

caggtggcgt acaagctcat gaaggagtac aaggccgacc agaagcaggc cttcctccag 1080

tttctgtcca agggctacaa cttcaaggat ctcaagaaca acatcgagtt caatctcgag 1140

ctgatcaaca atgagacagg cgaaaagacc accaagaagg tgaagatttc cctcttcaac 1200

gacatctccg aagagaactt taagaccatg ttcaccctca cgaacaagat ccagaagctc 1260

tccgccgagt tcaaccagac caagaacaag accacgaagg acgagatcac caagctgaag 1320

aaagagaagg tcaagtactt cttcgagggc gacaagctgt tcgcgttcaa agagtacggc 1380

accttctgca agtcctacaa gaaggtcgcc attgcctacg gcaggatcaa ggcggacatt 1440

aagtccctcg gcaaagaaaa ggtggacgcc gagaggacag attcctgggc tctcatcctt 1500

gagaacaagg acaacaagta cctcctcacg atcccgcgca acggcaacaa ggataagctc 1560

tccaaggcca agatctacat cgacggcctc cagaacgaga tcaacggcga gaactacctc 1620

tacaagttcg agtccctcac gctccgcgcg ctcgataagc tttgcttcgg gaaagagaac 1680

aacacgttcc gcaaggccat ctacctcgac cgcaagacgt tcccgaactt cagcaactcc 1740

gacggcaagc tcaaagagaa gttcgaattc aaagaggacg agaaagaact gattaacttc 1800

tacaagtacg tgctgaacct cgagagcacc cgcaagcaga tcgccctcaa gtatttcaac 1860

ggcttcgacg agttcgtgca gcgcgagttc gagacactgt ataagttcga aggggaactg 1920

aagaagatct gctacacaaa agagaagatt atcatctcga atgagacaaa ggacaagatc 1980

atcaacgact acaacgccaa gctctacaag atcacctcct acgacctcaa gaagaacgat 2040

cacgagtaca ttaaggacct cgagcagaag aagcagctcg acaggggcaa cccagagccg 2100

cataccaaga tctggcgcga tttcaggacc cggaacaacg aaacgaacca gtaccagacc 2160

aggctcaacc ctgaggtcaa gatctccttc gtcggcaagg acgaggactt tatcaaagag 2220

aacatcaagt tctccaacaa ccgcaggacc aaggaccgct acctcctgtc taccaccatc 2280

agccagaatt ccctcggccg gtacatcgac ctgaacttta aagaagaggc cgagatcgtc 2340

cgcttctacg aggaattcaa cctggacttc aacaagaatc agaagaatga cctcaactac 2400

ttctacggcc tcgaccgggg cgagaacgag cttgttacac tcggcatctt caacctcacg 2460

caaaagacca acgaggacaa gggcgtgcgc atcaaggttt tcaagctcaa gaacgaggct 2520

ttcctgttct ccgaccagta taacaaggac gaatccccga agctcgcgta caagaacctc 2580

agctactaca cctacgactc gcacccggac ctgtacgaaa ccgaggaagt gtcctgcatc 2640

gacctcacca cgtgcaagct tatcaagggc aatatctacc tgaacggcga catcggcacc 2700

tacctcaacc tcaagcttgt gtccgccaag cgcaagatct atggcatcgt gagcctcgga 2760

aagaacaagt ccgatatcat caagatcgag gatggctaca aggtggtgat taagggctac 2820

gacaaagagc gcggctccat cctctactcg ttcgacgaca agtacaagaa tatccagacc 2880

aaagaaaaaa tcatcgagat cctccaagag tacctcatta aggttaagaa caacctcacc 2940

gacaacgaga acattagcat cgaaaagatc aacaacctgc gcgactccat ctgcgccaac 3000

atggtgggcg ttatcgactc cctccagcaa aagcacccgg gcatgatctt tttcgaggac 3060

atggatatca actccaagaa cagcttcttc aatgagaaca atacgaccct cggcggcagg 3120

attgagcaga agctgctcca aaagctcagc tccctcaatc tcgtcccgcc gaactataag 3180

ctcatgccga cgctccagaa gaagattaag cagcttggca tcatcggcta cgtgaacaaa 3240

gagaatacct ccaacgcgtg cccgtactgc aagaagaatc tgttcggcca cggaaagggc 3300

tccaatttcg agaactccat gcaccactac aaagagagga acgggatcga ctacggcaac 3360

ttccttgagc aggccaagaa aacgaacaac gacaactgcg actaccacat gaagaacaac 3420

aactacggct tcgattacat caagtccggc gacgacctcg ccacctacaa tattgccaac 3480

cgcggaaaag aggacatcta cgacaaggtg aagcgcccga gagacaggca cgatggcgaa 3540

cttggaggta gaaagcgcgc caggtga 3567

<210> 9
<211> 4377
<212> DNA
<213> Artificial Sequence

<220>
<223> SpCas9 gene (plant codon optimized)

<400> 9
atggcaccga agaagaagcg caaggtgatg gacaagaagt acagcatcgg cctcgacatc 60

ggcaccaact cggtgggctg ggccgtcatc acggacgaat ataaggtccc gtcgaagaag 120

ttcaaggtcc tcggcaatac agaccgccac agcatcaaga aaaacttgat cggcgccctc 180

ctgttcgata gcggcgagac cgcggaggcg accaggctca agaggaccgc caggagacgg 240

tacactaggc gcaagaacag gatctgctac ctgcaggaga tcttcagcaa cgagatggcg 300

aaggtggacg actccttctt ccaccgcctg gaggaatcat tcctggtgga ggaggacaag 360

aagcatgagc ggcacccaat cttcggcaac atcgtcgacg aggtaagttt ctgcttctac 420

ctttgatata tatataataa ttatcattaa ttagtagtaa tataatattt caaatatttt 480

tttcaaaata aaagaatgta gtatatagca attgcttttc tgtagtttat aagtgtgtat 540

attttaattt ataacttttc taatatatga ccaaaacatg gtgatgtgca ggtggcctac 600

cacgagaagt acccgacaat ctaccacctc cggaagaaac tggtggacag cacagacaag 660

gcggacctcc ggctcatcta ccttgccctc gcgcatatga tcaagttccg cggccacttc 720

ctcatcgagg gcgacctgaa cccggacaac tccgacgtgg acaagctgtt catccagctc 780

gtgcagacgt acaatcaact gttcgaggag aaccccataa acgctagcgg cgtggacgcc 840

aaggccatcc tctcggccag gctctcgaaa tcaagaaggc tggagaacct tatcgcgcag 900

ttgccaggcg aaaagaagaa cggcctcttc ggcaacctta ttgcgctcag cctcggcctg 960

acgccgaact tcaaatcaaa cttcgacctc gcggaggacg ccaagctcca gctctcaaag 1020

gacacctacg acgacgacct cgacaacctc ctggcccaga taggagacca gtacgcggac 1080

ctcttcctcg ccgccaagaa cctctccgac gctatcctgc tcagcgacat ccttcgggtc 1140

aacaccgaaa ttaccaaggc accgctgtcc gccagcatga ttaaacgcta cgacgagcac 1200

catcaggacc tcacgctgct caaggcactc gtccgccagc agctccccga gaagtacaag 1260

gagatcttct tcgaccaatc aaaaaacggc tacgcgggat atatcgacgg cggtgccagc 1320

caggaagagt tctacaagtt catcaaacca atcctggaga agatggacgg caccgaggag 1380

ttgctggtca agctcaacag ggaggacctc ctcaggaagc agaggacctt cgacaacggc 1440

tccatcccgc atcagatcca cctgggcgaa ctgcatgcca tcctgcggcg ccaggaggac 1500

ttctacccgt tcctgaagga taaccgggag aagatcgaga agatcttgac gttccgcatc 1560

ccatactacg tgggcccgct ggctcgcggc aactcccggt tcgcctggat gacccggaag 1620

tcggaggaga ccatcacacc ctggaacttt gaggaggtgg tcgataaggg cgctagcgct 1680

cagagcttca tcgagcgcat gaccaacttc gataaaaacc tgcccaatga aaaagtcctc 1740

cccaagcact cgctgctcta cgagtacttc accgtgtaca acgagctcac caaggtcaaa 1800

tacgtcaccg agggcatgcg gaagccggcg ttcctgagcg gcgagcagaa gaaggcgata 1860

gtggacctcc tcttcaagac caacaggaag gtgaccgtga agcaattaaa agaggactac 1920

ttcaagaaaa tagagtgctt cgactccgtg gagatctcgg gcgtggagga tcggttcaac 1980

gcctcactcg gcacgtatca cgacctcctc aagatcatta aagacaagga cttcctcgac 2040

aacgaggaga acgaggacat cctcgaggac atcgtcctca ccctgaccct gttcgaggac 2100

cgcgaaatga tcgaggagag gctgaagacc tacgcgcacc tgttcgacga caaggtcatg 2160

aaacagctca agaggcgccg ctacactggt tggggaaggc tgtcccgcaa gctcattaat 2220

ggcatcaggg acaagcagag cggcaagacc atcctggact tcctcaagtc cgacgggttc 2280

gccaaccgca acttcatgca gctcattcac gacgactcgc tcacgttcaa ggaagacatc 2340

cagaaggcac aggtgagcgg gcagggtgac tccctccacg aacacatcgc caacctggcc 2400

ggctcgccgg ccattaaaaa gggcatcctg cagacggtca aggtcgtcga cgagctcgtg 2460

aaggtgatgg gccggcacaa gcccgaaaat atcgtcatag agatggccag ggagaaccag 2520

accacccaaa aagggcagaa gaactcgcgc gagcggatga aacggatcga ggagggcatt 2580

aaagagctcg ggtcccagat cctgaaggag caccccgtgg aaaataccca gctccagaat 2640

gaaaagctct acctctacta cctgcagaac ggccgcgaca tgtacgtgga ccaggagctg 2700

gacattaatc ggctatcgga ctacgacgtc gaccacatcg tgccgcagtc gttcctcaag 2760

gacgatagca tcgacaacaa ggtgctcacc cggtcggata aaaatcgggg caagagcgac 2820

aacgtgccca gcgaggaggt cgtgaagaag atgaaaaact actggcgcca gctcctcaac 2880

gcgaaactga tcacccagcg caagttcgac aacctgacga aggcggaacg cggtggcttg 2940

agcgaactcg ataaggcggg cttcataaaa aggcagctgg tcgagacgcg ccagatcacg 3000

aagcatgtcg cccagatcct ggacagccgc atgaatacta agtacgatga aaacgacaag 3060

ctgatccggg aggtgaaggt gatcacgctg aagtccaagc tcgtgtcgga cttccgcaag 3120

gacttccagt tctacaaggt ccgcgagatc aacaactacc accacgccca cgacgcctac 3180

ctgaatgcgg tggtcgggac cgccctgatc aagaagtacc cgaagctgga gtcggagttc 3240

gtgtacggcg actacaaggt ctacgacgtg cgcaaaatga tcgccaagtc cgagcaggag 3300

atcggcaagg ccacggcaaa atacttcttc tactcgaaca tcatgaactt cttcaagacc 3360

gagatcaccc tcgcgaacgg cgagatccgc aagcgcccgc tcatcgaaac caacggcgag 3420

acgggcgaga tcgtctggga taagggccgg gatttcgcga cggtccgcaa ggtgctctcc 3480

atgccgcaag tcaatatcgt gaaaaagacg gaggtccaga cgggcgggtt cagcaaggag 3540

tccatcctcc cgaagcgcaa ctccgacaag ctcatcgcga ggaagaagga ttgggacccg 3600

aaaaaatatg gcggcttcga cagcccgacc gtcgcataca gcgtcctcgt cgtggcgaag 3660

gtggagaagg gcaagtcaaa gaagctcaag tccgtgaagg agctgctcgg gatcacgatt 3720

atggagcggt cctccttcga gaagaacccg atcgacttcc tagaggccaa gggatataag 3780

gaggtcaaga aggacctgat tattaaactg ccgaagtact cgctcttcga gctggaaaac 3840

ggccgcaaga ggatgctcgc ctccgcaggc gagttgcaga agggcaacga gctcgccctc 3900

ccgagcaaat acgtcaattt cctgtacctc gctagccact atgaaaagct caagggcagc 3960

ccggaggaca acgagcagaa gcagctcttc gtggagcagc acaagcatta cctggacgag 4020

atcatcgagc agatcagcga gttctcgaag cgggtgatcc tcgccgacgc gaacctggac 4080

aaggtgctgt cggcatataa caagcaccgc gacaaaccaa tacgcgagca ggccgaaaat 4140

atcatccacc tcttcaccct caccaacctc ggcgctccgg cagccttcaa gtacttcgac 4200

accacgattg accggaagcg gtacacgagc acgaaggagg tgctcgatgc gacgctgatc 4260

caccagagca tcacagggct ctatgaaaca cgcatcgacc tgagccagct gggcggagac 4320

aagagaccac gggaccgcca cgatggcgag ctgggaggcc gcaagcgggc aaggtag 4377

<210> 10
<211> 466
<212> DNA
<213> Artificial Sequence

<220>
<223> sgRNAfor SpCas9

<400> 10
gaccaagccc gttattctga cagttctggt gctcaacaca tttatattta tcaaggagca 60

cattgttact cactgctagg agggaatcga actaggaata ttgatcagag gaactacgag 120

agagctgaag ataactgccc tctagctctc actgatctgg gtcgcatagt gagatgcagc 180

ccacgtgagt tcagcaacgg tctagcgctg ggcttttagg cccgcatgat cgggcttttg 240

tcgggtggtc gacgtgttca cgattgggga gagcaacgca gcagttcctc ttagtttagt 300

cccacctcgc ctgtccagca gagttctgac cggtttataa actcgcttgc tgcatcagac 360

ttgtgcggtc acgtaagtag ctggttttag agctagaaat agcaagttaa aataaggcta 420

gtccgttatc aacttgaaaa agtggcaccg agtcggtgct tttttt                466

<210> 11
<211> 414
<212> DNA
<213> Artificial Sequence

<220>
<223> crRNA for SmCmsI and Cps1

<400> 11
gaccaagccc gttattctga cagttctggt gctcaacaca tttatattta tcaaggagca      60

cattgttact cactgctagg agggaatcga actaggaata ttgatcagag gaactacgag     120

agagctgaag ataactgccc tctagctctc actgatctgg gtcgcatagt gagatgcagc     180

ccacgtgagt tcagcaacgg tctagcgctg ggcttttagg cccgcatgat cgggcttttg     240

tcgggtggtc gacgtgttca cgattgggga gagcaacgca gcagttcctc ttagtttagt     300

cccacctcgc ctgtccagca gagttctgac cggtttataa actcgcttgc tgcatcagac     360

ttgatttcta ctattgtaga tgatgcggtc acgtaagtag ctgaggcttt tttt           414

<210> 12
<211> 7
<212> PRT
<213> Artificial Sequence

<220>
<223> Cps1 RuvCI amino acid sequence

<400> 12
Tyr Gly Leu Asp Arg Gly Glu
1               5

<210> 13
<211> 7
<212> PRT
<213> Artificial Sequence

<220>
<223> Cps1 RuvCII amino acid sequence

<400> 13
Ile Phe Phe Glu Asp Met Asp
1               5

<210> 14
<211> 7
<212> PRT
<213> Artificial Sequence

<220>
<223> Cps1 RuvCIII amino acid sequence

<400> 14
Lys Ser Gly Asp Asp Leu Ala
1               5

<210> 15
<211> 7
<212> PRT
<213> Artificial Sequence

<220>
<223> Cps2 RuvCI amino acid sequence

<400> 15
Tyr Gly Leu Asp Arg Gly Glu
1               5

<210> 16
<211> 7
<212> PRT
<213> Artificial Sequence

<220>
<223> Cps2 RuvCII amino acid sequence

<400> 16
Ile Thr Leu Glu Asp Leu Thr
1               5

<210> 17
<211> 7
<212> PRT
<213> Artificial Sequence

<220>
<223> Cps2 RuvCIII amino acid sequence

<400> 17
Lys Ser Gly Asp Asp Leu Ala
1               5

<210> 18
<211> 7
<212> PRT
<213> Artificial Sequence

<220>
<223> SmCms1 RuvCI amino acid sequence

<400> 18
Tyr Gly Ile Asp Ala Gly Glu
1               5

<210> 19
<211> 7
<212> PRT
<213> Artificial Sequence

<220>
<223> SmCms1 RuvCII amino acid sequence

<400> 19
Ile Ser Ile Glu Asp Leu Lys
1               5

<210> 20
<211> 7

<212> PRT
<213> Artificial Sequence

<220>
<223> SmCms1 RuvCIII amino acid sequence

<400> 20
Asn Asp Pro Asp Lys Val Ala
1               5

<210> 21
<211> 7
<212> PRT
<213> Artificial Sequence

<220>
<223> ObCms1 RuvCI amino acid sequence

<400> 21
Phe Gly Ile Asp Arg Gly Asn
1               5

<210> 22
<211> 7
<212> PRT
<213> Artificial Sequence

<220>
<223> ObCms1 RuvCII amino acid sequence

<400> 22
Val Ala Leu Glu Asn Leu Ala
1               5

<210> 23
<211> 7
<212> PRT
<213> Artificial Sequence

<220>
<223> ObCms1 RuvCIII amino acid sequence

<400> 23
Asn Ser Pro Asp Thr Val Ala
1               5

<210> 24
<211> 7
<212> PRT
<213> Artificial Sequence

<220>
<223> MiCms1 RuvCI amino acid sequence

<400> 24
Tyr Gly Ile Asp Arg Gly Leu
1               5

<210> 25
<211> 7
<212> PRT
<213> Artificial Sequence

<220>
<223> MiCms1 RuvCII amino acid sequence

<400> 25
Ile Ala Leu Glu Asn Leu Asp
1               5

<210> 26
<211> 7
<212> PRT
<213> Artificial Sequence

<220>
<223> MiCms1 RuvCIII amino acid sequence

<400> 26
His Asn Ser Asp Asp Val Ala
1               5

<210> 27
<211> 14
<212> DNA
<213> Artificial Sequence

<220>
<223> Stem loop sequence

<220>
<221> variation
<222> 6,7,8
<223> /replace="a,c,g,t"

<400> 27
ucuacnnngu agau                                                    14


<210> 28
<211> 36
<212> DNA
<213> Artificial Sequence

<220>
<223> Cps1 stem loop sequence

<400> 28
ccttataact cctataagat ttctactatt gtagat                            36


<210> 29
<211> 36
<212> DNA
<213> Artificial Sequence

<220>
<223> Cps2 stem loop sequence

<400> 29
attgaaaacc aataataaat ttctactttt gtagat                            36


<210> 30
<211> 36
<212> DNA

<213> Artificial Sequence

<220>
<223> Cms1_Sm stem loop sequence

<400> 30
gtccaaagga cggattaaat ttctactatt gtagat                                36


<210> 31
<211> 36
<212> DNA
<213> Artificial Sequence

<220>
<223> Cms1_Mi stem loop sequence

<400> 31
gtctaaaccg acccaataat ttctactgtt gtagat                                36


<210> 32
<211> 36
<212> DNA
<213> Artificial Sequence

<220>
<223> Cms1_Ob stem loop sequence

<400> 32
atcgaacaga ctcataaaat ttctactgtt gtagat                                36


<210> 33
<211> 95
<212> DNA
<213> Artificial Sequence

<220>
<223> Mutant GUS gene sequence

<400> 33
gggcaggcca gcgtatcgtg ctgcgtttcg atgcggtcac gtaagtagct gaggcgggca      60

ggccagcgta tcgtgctgcg tttcgatgcg gtcac                                 95


<210> 34
<211> 40
<212> DNA
<213> Triticum aestivum

<220>
<223> Repaired GUS gene sequence

<400> 34
gggcaggcca gcgtatcgtg ctgcgtttcg atgcggtcac                            40


<210> 35
<211> 15
<212> DNA
<213> Artificial Sequence

<220>
<223> Stem loop sequence

<220>
<221> variation
<222> 6,7,8,9
<223> /replace="a,c,g,t"

<400> 35
ucuacnnnng uagau                                                        15


<210> 36
<211> 16
<212> DNA
<213> Artificial Sequence

<220>
<223> Stem loop sequence

<220>
<221> variation
<222> 6,7,8,9,10
<223> /replace="a,c,g,t"

<400> 36
ucuacnnnnn guagau                                                       16


<210> 37
<211> 19
<212> DNA
<213> Artificial Sequence

<220>
<223> Class 2 Type V consensus stem loop sequence

<220>
<221> variation
<222> 1,11
<223> /replace="a,c,g,t"

<400> 37
natttctact nttgtagat                                                    19


<210> 38
<211> 19
<212> DNA
<213> Artificial Sequence

<220>
<223> Cps1 stem loop sequence 5' handle

<400> 38
gatttctact attgtagat                                                    19


<210> 39
<211> 19
<212> DNA
<213> Artificial Sequence

<220>
<223> Cps2 stem loop sequence 5′ handle

<400> 39
aatttctact tttgtagat                                                    19